(19) Europäisches Patentamt
European Patent Office
Office européen des brevets

(11) EP 2 572 199 B1

(12) **EUROPEAN PATENT SPECIFICATION**

(45) Date of publication and mention of the grant of the patent:
**08.03.2017 Bulletin 2017/10**

(21) Application number: **11784238.5**

(22) Date of filing: **19.05.2011**

(51) Int Cl.:
***G01N 33/574*** *(2006.01)* ***A61K 31/4155*** *(2006.01)*
***A61K 31/517*** *(2006.01)*

(86) International application number:
**PCT/US2011/037141**

(87) International publication number:
**WO 2011/146710 (24.11.2011 Gazette 2011/47)**

(54) **COMBINATION**

KOMBINATION

COMBINAISON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.05.2010 US 34699210 P**

(43) Date of publication of application:
**27.03.2013 Bulletin 2013/13**

(73) Proprietor: **Novartis AG**
**4056 Basel (CH)**

(72) Inventors:
- **GILMER, Tona, M.**
**Research Triangle Park**
**NC 27709 (US)**
- **KUMAR, Rakesh**
**Collegeville**
**PA 19426 (US)**

(74) Representative: **Rudge, Sewkian et al**
**Novartis Pharma AG**
**Patent Department**
**4002 Basel (CH)**

(56) References cited:
**WO-A1-2011/060380 WO-A2-2008/063853**
**US-A1- 2007 015 775 US-A1- 2010 041 726**

- **J. KORKOLA ET AL: "Abstract A179: Synergistic interactions of Lapatinib and an AKT inhibitor in HER2 positive breast cancer cell lines depends on PI3K pathway status", MOLECULAR CANCER THERAPEUTICS, vol. 8, no. Supplement 1, 10 December 2009 (2009-12-10), pages A179-A179, XP055078200, ISSN: 1535-7163, DOI: 10.1158/1535-7163.TARG-09-A179**
- **H. HIRAI ET AL: "MK-2206, an Allosteric Akt Inhibitor, Enhances Antitumor Efficacy by Standard Chemotherapeutic Agents or Molecular Targeted Drugs In vitro and In vivo", MOLECULAR CANCER THERAPEUTICS, vol. 9, no. 7, 22 June 2010 (2010-06-22), pages 1956-1967, XP055074270, ISSN: 1535-7163, DOI: 10.1158/1535-7163.MCT-09-1012**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 572 199 B1

## Description

FIELD OF THE INVENTION

The present invention relates to combinations useful in a method of treating cancer in a mammal. In particular, the method relates to a novel combination comprising the dual EGF-R/erbB-2 inhibitor: N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, and the Akt inhibitor: *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof, pharmaceutical compositions comprising the same, and such combinations for use in the treatment of cancer.

BACKGROUND OF THE INVENTION

Generally, cancer results from the deregulation of the normal processes that control cell division, differentiation and apoptotic cell death. Apoptosis (programmed cell death) plays essential roles in embryonic development and pathogenesis of various diseases, such as degenerative neuronal diseases, cardiovascular diseases and cancer. One of the most commonly studied pathways, which involves kinase regulation of apoptosis, is cellular signaling from growth factor receptors at the cell surface to the nucleus (Crews and Erikson, Cell, 74:215-17, 1993).

Protein tyrosine kinases (PTKs) catalyze the phosphorylation of specific tyrosyl residues in various proteins involved in the regulation of cell growth and differentiation. (A.F. Wilks, Progress in Growth Factor Research, 1990, 2, 97-111; S.A. Courtneidge, Dev. Supp.I, 1993, 57-64; J.A. Cooper, Semin. Cell Biol., 1994, 5(6), 377-387; R.F. Paulson, Semin. Immunol., 1995, 7(4), 267-277; A.C. Chan, Curr. Opin. Immunol., 1996, 8(3), 394-401). Inappropriate or uncontrolled activation of many PTKs, i.e. aberrant PTK activity, for example by over-expression or mutation, has been shown to result in uncontrolled cell growth.

Aberrant protein tyrosine kinase (PTK) activity has been implicated in a variety of disorders including psoriasis, rheumatoid arthritis, bronchitis, as well as cancer. Development of effective treatments for such disorders is a constant and ongoing enterprise in the medical field. The ErbB family of PTKs, which includes ErbB-2, EGFR, ErbB-3 and ErbB-4, is one group of PTKs that has attracted interest as a therapeutic target. Currently, of special interest, is the role of ErbB family PTKs in hyperproliferative disorders, particularly human malignancies. Elevated EGFR activity has, for example, been implicated in non-small cell lung, bladder, and head and neck cancers. Furthermore, increased ErbB-2 activity has been implicated in breast, ovarian, gastric and pancreatic cancers. Overexpression of HRG and/or HER3 has been reported in numerous cancers, including gastric, ovarian, prostate, bladder, and breast tumors and is associated with poor prognosis (B.Tanner,J Clin Oncol. 2006, 24(26):4317-23; M. Hayashi, Clin. Cancer Res. 2008.14(23):7843-9.; H. Kaya, Eur J Gynaecol Oncol. 2008;29(4):350-6;). Consequently, inhibition of ErbB family PTKs should provide a treatment for disorders characterized by aberrant ErbB family PTK activity. The biological role of ErbB family PTKs and their implication in various disease states is discussed, for instance in U.S. patent 5,773,476; International Patent Application WO 99/35146; M.C. Hung et al, Seminars in Oncology, 26: 4, Suppl. 12 (August) 1999, 51-59; Ullrich et al, Cell, 61: 203-212, April 20, 1990; Modjtahedi et al, Int'l. J. of Oncology, 13: 335-342,1998; and J.R. Woodburn, Pharmacol. Ther., 82: 2-3, 241-250, 1999, it is generally accepted that inhibitors of ErbB family kinases will be useful for the treatment of such cancers or other condition associated with ErbB family kinases.

Apoptosis (programmed cell death) plays essential roles in embryonic development and pathogenesis of various diseases, such as degenerative neuronal diseases, cardiovascular diseases and cancer. Recent work has led to the identification of various pro- and anti-apoptotic gene products that are involved in the regulation or execution of programmed cell death. Expression of anti-apoptotic genes, such as Bcl2 or Bcl-$x_L$, inhibits apoptotic cell death induced by various stimuli. On the other hand, expression of pro-apoptotic genes, such as Bax or Bad, leads to programmed cell death (Adams et al. Science, 281:1322-1326 (1998)). The execution of programmed cell death is mediated by caspase -1 related proteinases, including caspase-3, caspase- 7, caspase-8 and caspase-9 etc (Thornberry et al. Science, 281:1312-1316 (1998)).

The phosphatidylinositol 3'-OH kinase (PI3K)/Akt/PKB pathway appears important for regulating cell survival/cell death (Kulik et al. Mol.Cell.Biol. 17:1595-1606 (1997); Franke et al, Cell, 88:435-437 (1997); Kauffmann-Zeh et al. Nature 385:544-548 (1997) Hemmings Science, 275:628-630 (1997); Dudek et al., Science, 275:661-665 (1997)). Survival factors, such as platelet derived growth factor (PDGF), nerve growth factor (NGF) and insulin-like growth factor-1 (IGF-I), promote cell survival under various conditions by inducing the activity of PI3K (Kulik et al. 1997, Hemmings 1997). Activated PI3K leads to the production of phosphatidylinositol (3,4,5)-triphosphate (PtdIns (3,4,5)-P3), which in turn binds to, and promotes the activation of, the serine/threonine kinase Akt, which contains a pleckstrin homology (PH)-domain (Franke et al Cell, 81:727-736 (1995); Hemmings Science, 277:534 (1997); Downward, Curr. Opin. Cell Biol. 10:262-267 (1998), Alessi et al., EMBO J. 15: 6541-6551 (1996)). Specific inhibitors of PI3K or dominant negative Akt/PKB mutants abolish survival-promoting activities of these growth factors or cytokines. It has been previously disclosed that inhibitors

of PI3K (LY294002 or wortmannin) blocked the activation of Akt/PKB by upstream kinases. In addition, introduction of constitutively active PI3K or Akt/PKB mutants promotes cell survival under conditions in which cells normally undergo apoptotic cell death (Kulik et al. 1997, Dudek et al. 1997).

Analysis of Akt levels in human tumors showed that Akt2 is overexpressed in a significant number of ovarian (J. Q. Cheung et al. Proc. Natl. Acad. Sci. U.S.A. 89:9267-9271(1992)) and pancreatic cancers (J. Q. Cheung et al. Proc. Natl. Acad. Sci. U.S.A. 93:3636-3641 (1996)). Similarly, Akt3 was found to be overexpressed in breast and prostate cancer cell lines (Nakatani et al. J. Biol.Chem. 274:21528-21532 (1999). It was demonstrated that Akt-2 was over-expressed in 12% of ovarian carcinomas and that amplification of Akt was especially frequent in 50% of undifferentiated tumors, suggestion that Akt may also be associated with tumor aggressiveness (Bellacosa, et al., Int. J. Cancer, 64, pp. 280-285, 1995). Increased Akt1 kinase activity has been reported in breast, ovarian and prostate cancers (Sun et al. Am. J. Pathol. 159: 431-7 (2001)).

The tumor suppressor PTEN, a protein and lipid phosphatase that specifically removes the 3' phosphate of PtdIns(3,4,5)-P3, is a negative regulator of the PI3K/Akt pathway (Li et al. Science 275:1943-1947 (1997), Stambolic et al. Cell 95:29-39 (1998), Sun et al. Proc. Nati. Acad. Sci. U.S.A. 96:6199-6204 (1999)). Germline mutations of PTEN are responsible for human cancer syndromes such as Cowden disease (Liaw et al. Nature Genetics 16:64-67 (1997)). PTEN is deleted in a large percentage of human tumors and tumor cell lines without functional PTEN show elevated levels of activated Akt (Li et al. supra, Guldberg et al. Cancer Research 57:3660-3663 (1997), Risinger et al. Cancer Research 57:4736-4738 (1997)).

These observations demonstrate that the PI3K/Akt pathway plays important roles for regulating cell survival or apoptosis in tumorigenesis and/or cancer.

WO 2008/063853, Korkola et al (2009) Mol. Cancer Ther. 8, A179; Hirai et al, (June 2010), Mol. Cancer Ther. 9, 1956-1967 and WO2011/060380 describe a combination of lapatinib and an Akt-inhibitor.

US2007/015775 and US 2010/041726 disclose Compounds of structure (I) and (II) respectively.

It would be useful to provide a novel therapy which provides more effective and/or enhanced treatment of an individual suffering the effects of cancer.

SUMMARY OF THE INVENTION

One embodiment of this invention provides a combination comprising:

(i) a compound of Structure (I):

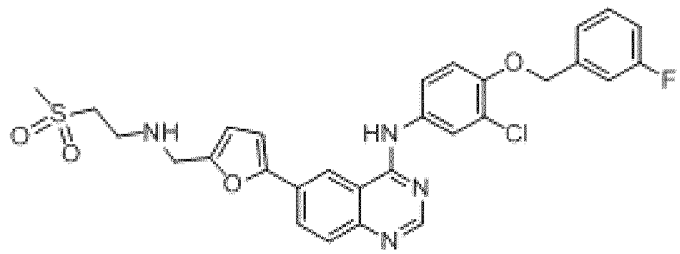

(I)

or a pharmaceutically acceptable hydrate and/or salt thereof; and

(ii) a compound of Structure (II):

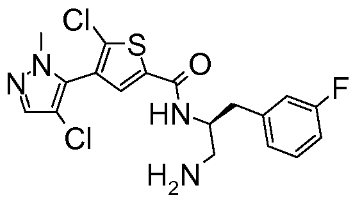

(II)

or a pharmaceutically acceptable salt thereof.

There is also described a method of treating cancer in a human in need thereof which comprises the in vivo administration of a therapeutically effective amount of a combination of N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human.

There is also described a method of treating cancer in a human in need thereof which comprises the in vivo administration of a therapeutically effective amount of a combination of N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human,

wherein the combination is administered within a specified period, and
wherein the combination is administered for a duration of time.

There is also described a method of treating cancer in a human in need thereof which comprises the in vivo administration of a therapeutically effective amount of a combination of N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, to such human,

wherein the compounds of the combination are administered sequentially.

BRIEF DESCRIPTION OF THE DRAWINGS

Figure - 1 Figure 1 depicts the dose response curves of cell growth inhibition by Compound A, Compound B or the 1 to 1 molar ratio combination of Compound A and Compound B in BT474, KPL-4 and BT474-J4 cells.
Figure - 2 Figure 2 depicts the dose response curves of apoptosis induction by Compound A, Compound B or the 10 to 1 molar ratio combination of Compound A and Compound B in BT474, KPL-4 and BT474-J4 cells.

DETAILED DESCRIPTION OF THE INVENTION

The present invention relates to combinations that exhibit antiproliferative activity. Suitably, the method relates to methods of treating cancer by the co-administration of N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, (hereinafter Compound A, or a pharmaceutically acceptable hydrate and/or salt, suitably the ditosylate monohydrate salt, thereof,
which compound is represented by Structure I:

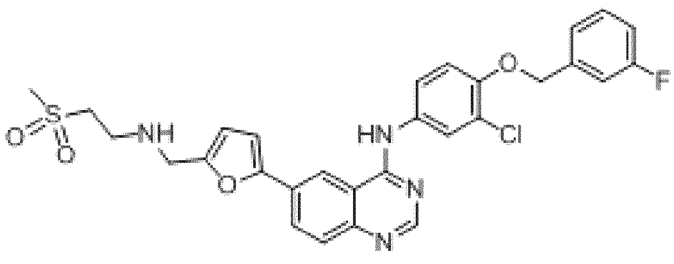

(I));

and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, (hereinafter Compound B or a pharmaceutically acceptable salt, suitably the hydrochloride salt, thereof,
which compound is represented by Structure II:

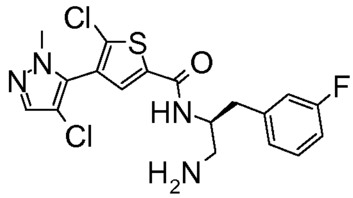

(II)).

Compound A is disclosed and claimed, along with pharmaceutically acceptable solvates and salts thereof, as being useful as an inhibitor of EGF-R/erbB-2 activity, particularly in treatment of cancer, in International Application No. PCT/EP99/00048, having an International filing date of January 8, 1999, International Publication Number WO 99/35146 and an International Publication date of July 15, 1999 and in US2007/015775, Compound A is the compound of Example 29. Compound A can be prepared as described in International Application No. PCT/EP99/00048.

Suitably, Compound A is in the form of a ditosylate monohydrate salt. This salt form can be prepared by one of skill in the art from the description in International Application No. PCT/US01/20706, having an International filing date of June 28, 2001, International Publication Number WO 02/02552 and an International Publication date of January 10, 2002, see particularly Example 10.

Suitable pharmaceutical compositions containing Compound A as a single active ingredient are prepared as described in International Application No. PCT/US2006/014447, having an International filing date of April 18, 2006, International Publication Number WO 06/113649 and an International Publication date of October 26, 2006, see particularly the formulation in Table 3.

Compound A is sold commercially as the ditosylate monohydrate salt and is known by the generic name lapatinib and trade names Tykerb® and Tyverb®.

Compound B is disclosed and claimed, along with pharmaceutically acceptable salts thereof, as being useful as an inhibitor of AKT activity, particularly in treatment of cancer, in International Application No. PCT/US2008/053269, having an International filing date of February 7, 2008; International Publication Number WO 2008/098104 and an International Publication date of August 14, . Compound B is the compound of example 96. Compound B is also disclosed in US 2010/041726. Compound B can be prepared as described in International Application No. PCT/US2008/053269.

Suitably, Compound B is in the form of a hydrochloride salt. The salt form can be prepared by one of skill in the art from the description in International Application No. PCT/US2010/022323, having an International filing date of January 28, 2010; International Publication Number WO 2010/088331 and an International Publication date of August 5, 2010. Compound B in the form of a hydrochloride salt can be prepared as described in International Application No. PCT/US2010/022323.

The administration of a therapeutically effective amount of the combinations of the invention are advantageous over the individual component compounds in that the combinations will provide one or more of the following improved properties when compared to the individual administration of a therapeutically effective amount of a component compound: i) a greater anticancer effect than the most active single agent, ii) synergistic or highly synergistic anticancer activity, iii) a dosing protocol that provides enhanced anticancer activity with reduced side effect profile, iv) a reduction in the toxic effect proflie, v) an increase in the therapeutic window, or vi) an increase in the bioavailability of one or both of the component compounds.

The compounds of the combination of the invention may contain one or more chiral atoms, or may otherwise be capable of existing as two enantiomers. Accordingly, the compounds of this invention include mixtures of enantiomers as well as purified enantiomers or enantiomerically enriched mixtures. Also, it is understood that all tautomers and mixtures of tautomers are included within the scope of Compound A, and pharmaceutically acceptable hydrates and/or salts thereof, and Compound B, and pharmaceutically acceptable salts thereof.

The compounds of the combination of the invention may form a solvate which is understood to be a complex of variable stoichiometry formed by a solute (in this invention, Compound A or a salt thereof and/or Compound B or a salt thereof) and a solvent. Such solvents for the purpose of the invention may not interfere with the biological activity of the solute. Examples of suitable solvents include, but are not limited to, water, methanol, dimethyl sulfoxide, ethanol and acetic acid. Suitably the solvent used is a pharmaceutically acceptable solvent. Suitably the solvent used is water.

The pharmaceutically acceptable salts of the compounds of the invention are readily prepared by those of skill in the art.

Also, contemplated herein is a method of treating cancer using a combination of the invention where Compound A, or a pharmaceutically acceptable hydrate and/or salt thereof, and/or Compound B or a pharmaceutically acceptable salt thereof are administered as pro-drugs. Pharmaceutically acceptable pro-drugs of the compounds of the invention are readily prepared by those of skill in the art.

When referring to a dosing protocol, the term "day", "per day" and the like, refer to a time within one calendar day which begins at midnight and ends at the following midnight.

By the term "treating" and derivatives thereof as used herein, is meant therapeutic therapy. In reference to a particular condition, treating means: (1) to ameliorate or prevent the condition of one or more of the biological manifestations of the condition, (2) to interfere with (a) one or more points in the biological cascade that leads to or is responsible for the condition or (b) one or more of the biological manifestations of the condition, (3) to alleviate one or more of the symptoms, effects or side effects associated with the condition or treatment thereof, or (4) to slow the progression of the condition or one or more of the biological manifestations of the condition. Prophylactic therapy is also contemplated thereby. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof. Prophylactic therapy is appropriate, for example, when a subject is considered at high risk for developing cancer, such as when a subject has a strong family history of cancer or when a subject has been exposed to a carcinogen.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

By the term "combination" and derivatives thereof, as used herein is meant either, simultaneous administration or any manner of separate sequential administration of a therapeutically effective amount of Compound A, or a pharmaceutically acceptable hydrate and/or salt thereof, and Compound B or a pharmaceutically acceptable salt thereof. Preferably, if the administration is not simultaneous, the compounds are administered in a close time proximity to each other. Furthermore, it does not matter if the compounds are administered in the same dosage form, e.g. one compound may be administered topically and the other compound may be administered orally. Suitably, both compounds are administered orally.

By the term "combination kit" as used herein is meant the pharmaceutical composition or compositions that are used to administer Compound A, or a pharmaceutically acceptable hydrate and/or salt thereof, and Compound B, or a pharmaceutically acceptable salt thereof, according to the invention. When both compounds are administered simultaneously, the combination kit can contain Compound A, or a pharmaceutically acceptable hydrate and/or salt thereof, and Compound B, or a pharmaceutically acceptable salt thereof, in a single pharmaceutical composition, such as a tablet, or in separate pharmaceutical compositions. When the compounds are not administered simultaneously, the combination kit will contain Compound A, or a pharmaceutically acceptable hydrate and/or salt thereof, and Compound B, or a pharmaceutically acceptable salt thereof, in separate pharmaceutical compositions. The combination kit can comprise Compound A, or a pharmaceutically acceptable hydrate and/or salt thereof, and Compound B, or a pharmaceutically acceptable salt thereof, in separate pharmaceutical compositions in a single package or in separate pharmaceutical compositions in separate packages.

In one aspect there is provided a combination kit comprising the components:

Compound A, or a pharmaceutically acceptable hydrate and/or salt thereof, in association with a pharmaceutically acceptable carrier; and
Compound B, or a pharmaceutically acceptable salt thereof, in association with a pharmaceutically acceptable carrier.

In one embodiment of the invention the combination kit comprises the following components:

Compound A, or a pharmaceutically acceptable hydrate and/or salt thereof, in association with a pharmaceutically acceptable carrier; and
Compound B, or a pharmaceutically acceptable salt thereof, in association with a pharmaceutically acceptable carrier,

wherein the components are provided in a form which is suitable for sequential, separate and/or simultaneous administration.

In one embodiment the combination kit comprises:

a first container comprising Compound A, or a pharmaceutically acceptable hydrate and/or salt thereof, in association with a pharmaceutically acceptable carrier; and
a second container comprising Compound B, or a pharmaceutically acceptable salt thereof, in association with a pharmaceutically acceptable carrier, and a container means for containing said first and second containers.

The "combination kit" can also be provided by instruction, such as dosage and administration instructions. Such dosage and administration instructions can be of the kind that is provided to a doctor, for example by a drug product

label, or they can be of the kind that is provided by a doctor, such as instructions to a patient.

Unless otherwise defined, in all dosing protocols described herein, the regimen of compounds administered does not have to commence with the start of treatment and terminate with the end of treatment, it is only required that the number of consecutive days in which both compounds are administered and the optional number of consecutive days in which only one of the component compounds is administered, or the indicated dosing protocol - including the amount of compound administered, occur at some point during the course of treatment.

As used herein the term "Compound $A^2$" means ---Compound A, or a pharmaceutically acceptable hydrate and/or salt thereof---.

As used herein the term "Compound $B^2$" means ---Compound B, or a pharmaceutically acceptable salt thereof---.

In one reference embodiment of the present disclosure Compound B is replaced by:

8-[4-(1-aminocyclobutyl)phenyl]-9-phenyl[1,2,4]triazolo[3,4-f]-1,6-naphthyridin-3(2H)-one; which has the following structure (depicted as the chloride salt):

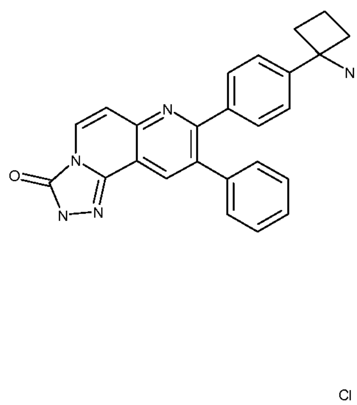
;

provided that when said compound is administered on a day 1, day 3, and day 5 of a dosing protocol the compound is not administered at a dose selected from: 30mg, 45mg or 60mg.

In one reference embodiment of the present disclosure Compound $B^2$ is replaced by the compound:

8-[4-(1-aminocyclobutyl)phenyl]-9-phenyl[1,2,4]triazolo[3,4-f]-1,6-naphthyridin-3(2H)-one or a pharmaceutically acceptable salt thereof;
provided that when said compound is administered on a day 1, day 3, and day 5 of a dosing protocol the compound is not administered at a dose selected from: 30mg, 45mg or 60mg.

The compound 8-[4-(1-aminocyclobutyl)phenyl]-9-phenyl[1,2,4]triazolo[3,4-f]-1,6-naphthyridin-3(2H)-one is disclosed and claimed, along with pharmaceutically acceptable salts thereof, as being useful as an inhibitor of AKT activity, particularly in treatment of cancer, in United States Patent 7,576,209 which issued on August 18, 2009. 8-[4-(1-aminocyclobutyl)phenyl]-9-phenyl[1,2,4]triazolo[3,4-f]-1,6-naphthyridin-3(2H)-one can be prepared as described in United States Patent 7,576,209.

The term "loading dose" as used herein will be understood to mean a single dose or short duration regimen of Compound A or Compound B having a dosage higher than the maintenance dose administered to the subject to rapidly increase the blood concentration level of the drug. Suitably, a short duration regimen for use herein will be from: 1 to 14 days; suitably from 1 to 7 days; suitably from 1 to 3 days; suitably for three days; suitably for two days; suitably for one day. In some embodiments, the "loading dose" can increase the blood concentration of the drug to a therapeutically effective level. In some embodiments, the "loading dose" can increase the blood concentration of the drug to a therapeutically effective level in conjunction with a maintenance dose of the drug. The "loading dose" can be administered once per day, or more than once per day (e.g., up to 4 times per day). Suitably the "loading dose" will be administered once a day. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading dose will be administered for from 1 to 7 days; suitably from 1 to 5 days; suitably from 1 to 3 days; suitably for 1 day; suitably for 2 days; suitably for 3 days, followed by a maintenance dosing protocol.

The term "maintenance dose" as used herein will be understood to mean a dose that is serially administered (for example, at least twice), and which is intended to either slowly raise blood concentration levels of the compound to

a therapeutically effective level, or to maintain such a therapeutically effective level. The maintenance dose is generally administered once per day and the daily dose of the maintenance dose is lower than the total daily dose of the loading dose.

Suitably the combinations of this invention are administered within a "specified period".

By the term "specified period" and derivatives thereof, as used herein is meant the interval of time between the administration of one of Compound $A^2$ and Compound $B^2$ and the other of Compound $A^2$ and Compound $B^2$. Unless otherwise defined, the specified period can include simultaneous administration. When both compounds of the invention are administered once a day the specified period refers to timing of the administration of Compound $A^2$ and Compound $B^2$ during a single day. When one or both compounds of the invention are administered more than once a day, the specified period is calculated based on the first administration of each compound on a specific day. All administrations of a compound of the invention that are subsequent to the first during a specific day are not considered when calculating the specific period.

Suitably, if the compounds are administered within a "specified period" and not administered simultaneously, they are both administered within about 24 hours of each other - in this case, the specified period will be about 24 hours; suitably they will both be administered within about 12 hours of each other - in this case, the specified period will be about 12 hours; suitably they will both be administered within about 11 hours of each other - in this case, the specified period will be about 11 hours; suitably they will both be administered within about 10 hours of each other - in this case, the specified period will be about 10 hours; suitably they will both be administered within about 9 hours of each other-in this case, the specified period will be about 9 hours; suitably they will both be administered within about 8 hours of each other - in this case, the specified period will be about 8 hours; suitably they will both be administered within about 7 hours of each other-in this case, the specified period will be about 7 hours; suitably they will both be administered within about 6 hours of each other - in this case, the specified period will be about 6 hours; suitably they will both be administered within about 5 hours of each other-in this case, the specified period will be about 5 hours; suitably they will both be administered within about 4 hours of each other - in this case, the specified period will be about 4 hours; suitably they will both be administered within about 3 hours of each other-in this case, the specified period will be about 3 hours; suitably they will be administered within about 2 hours of each other - in this case, the specified period will be about 2 hours; suitably they will both be administered within about 1 hour of each other - in this case, the specified period will be about 1 hour. As used herein, the administration of Compound $A^2$ and Compound $B^2$ in less than about 45 minutes apart is considered simultaneous administration.

Suitably, when the combination of the invention is administered for a "specified period", the compounds will be co-administered for a "duration of time".

By the term "duration of time" and derivatives thereof, as used herein is meant that both compounds of the invention are administered within a "specified period" for an indicated number of consecutive days, optionally followed by a number of consecutive days where only one of the component compounds is administered.

Regarding "specified period" administration:

Suitably, during the course of treatment, both compounds will be administered within a specified period for at least 1 day - in this case, the duration of time will be at least 1 day; suitably, during the course of treatment, both compounds will be administered within a specified period for at least 2 consecutive days - in this case, the duration of time will be at least 2 days; suitably, during the course of treatment, both compounds will be administered within a specified period for at least 3 consecutive days - in this case, the duration of time will be at least 3 days; suitably, during the course of treatment, both compounds will be administered within a specified period for at least 5 consecutive days - in this case, the duration of time will be at least 5 days; suitably, during the course of treatment, both compounds will be administered within a specified period for at least 7 consecutive days-in this case, the duration of time will be at least 7 days; suitably, during the course of treatment, both compounds will be administered within a specified period for at least 14 consecutive days - in this case, the duration of time will be at least 14 days; suitably, during the course of treatment, both compounds will be administered within a specified period for at least 30 consecutive days - in this case, the duration of time will be at least 30 days. When, during the course of treatment, both compounds are administered within a specified period for over 30 days, the treatment is considered chronic treatment and will continue until an altering event, such as a reassessment in cancer status or a change in the condition of the patient, warrants a modification to the protocol.

Further regarding "specified period" administration:

Suitably, during the course of treatment, both compounds will be administered within a specified period for at least 1 day, followed by the administration of Compound $A^2$ alone for at least 1 day - in this case, the duration of time will be at least 2 days; suitably, during the course of treatment, both compounds will be administered within a specified period for at least 1 day, followed by administration of Compound $A^2$ alone for at least 2 days - in this case, the duration

of time will be at least 3 days; suitably, during the course of treatment, both compounds will be administered within a specified period for at least 1 day, followed by administration of Compound $A^2$ alone for at least 3 days - in this case, the duration of time will be at least 4 days; suitably, during the course of treatment, both compounds will be administered within a specified period for at least 1 day, followed by administration of Compound $A^2$ alone for at least 4 days - in this case, the duration of time will be at least 5 days; suitably, during the course of treatment, both compounds will be administered within a specified period for at least 1 day, followed by administration of Compound $A^2$ alone for at least 5 days - in this case, the duration of time will be at least 6 days; suitably, during the course of treatment, both compounds will be administered within a specified period for at least 1 day, followed by administration of Compound $A^2$ alone for at least 6 days - in this case, the duration of time will be at least 7 days; suitably, during the course of treatment, both compounds will be administered within a specified period for at least 1 day, followed by administration of Compound $A^2$ alone for at least 7 days - in this case, the duration of time will be at least 8 days; suitably, during the course of treatment, both compounds will be administered within a specified period for at least 2 consecutive days, followed by administration of Compound $A^2$ alone for at least 1 day - in this case, the duration of time will be at least 3 days; suitably, during the course of treatment, both compounds will be administered within a specified period for at least 2 consecutive days, followed by administration of Compound $A^2$ alone for at least 2 consecutive days - in this case, the duration of time will be at least 4 days; suitably, during the course of treatment, both compounds will be administered within a specified period for at least 2 consecutive days, followed by administration of Compound $A^2$ alone for at least 3 consecutive days - in this case, the duration of time will be at least 5 days; suitably, during the course of treatment, both compounds will be administered within a specified period for at least 2 consecutive days, followed by administration of Compound $A^2$ alone for at least 4 consecutive days - in this case, the duration of time will be at least 6 days; suitably, during the course of treatment, both compounds will be administered within a specified period for at least 2 consecutive days, followed by administration of Compound $A^2$ alone for at least 5 consecutive days - in this case, the duration of time will be at least 7 days; suitably, during the course of treatment, both compounds will be administered within a specified period for at least 2 consecutive days, followed by administration of Compound $A^2$ alone for at least 6 consecutive days - in this case, the duration of time will be at least 8 days; suitably, during the course of treatment, both compounds will be administered within a specified period for at least 2 consecutive days, followed by administration of Compound $A^2$ alone for at least 7 consecutive days - in this case, the duration of time will be at least 9 days; suitably, during the course of treatment, both compounds will be administered within a specified period for at least 3 consecutive days, followed by administration of Compound $A^2$ alone for at least 1 day - in this case, the duration of time will be at least 4 days; suitably, during the course of treatment, both compounds will be administered within a specified period for at least 3 consecutive days, followed by administration of Compound $A^2$ alone for at least 2 consecutive days - in this case, the duration of time will be at least 5 days; suitably, during the course of treatment, both compounds will be administered within a specified period for at least 3 consecutive days, followed by administration of Compound $A^2$ alone for at least 3 consecutive days - in this case, the duration of time will be at least 6 days; suitably, during the course of treatment, both compounds will be administered within a specified period for at least 3 consecutive days, followed by administration of Compound $A^2$ alone for at least 4 consecutive days - in this case, the duration of time will be at least 7 days; suitably, during the course of treatment, both compounds will be administered within a specified period for at least 3 consecutive days, followed by administration of Compound $A^2$ alone for at least 5 consecutive days - in this case, the duration of time will be at least 8 days; suitably, during the course of treatment, both compounds will be administered within a specified period for at least 3 consecutive days, followed by administration of Compound $A^2$ alone for at least 6 consecutive days - in this case, the duration of time will be at least 9 days; suitably, during the course of treatment, both compounds will be administered within a specified period for at least 3 consecutive days, followed by administration of Compound $A^2$ alone for at least 7 consecutive days - in this case, the duration of time will be at least 10 days; suitably, during the course of treatment, both compounds will be administered within a specified period for at least 4 consecutive days, followed by administration of Compound $A^2$ alone for at least 1 day - in this case, the duration of time will be at least 5 consecutive days; suitably, during the course of treatment, both compounds will be administered within a specified period for at least 4 consecutive days, followed by administration of Compound $A^2$ alone for at least 2 consecutive days - in this case, the duration of time will be at least 6 consecutive days; suitably, during the course of treatment, both compounds will be administered within a specified period for at least 4 consecutive days, followed by administration of Compound $A^2$ alone for at least 3 consecutive days - in this case, the duration of time will be at least 7 consecutive days; suitably, during the course of treatment, both compounds will be administered within a specified period for at least 4 consecutive days, followed by administration of Compound $A^2$ alone for at least 4 consecutive days - in this case, the duration of time will be at least 8 consecutive days; suitably, during the course of treatment, both compounds will be administered within a specified period for at least 4 consecutive days, followed by administration of Compound $A^2$ alone for at least 7 consecutive days - in this case, the duration of time will be at least 11 consecutive days; suitably, during the course of treatment, both compounds will be administered within a specified period for at least 5 consecutive days, followed by administration of Compound $A^2$ alone for at least 1 day - in this case, the duration of time will be at least 6 consecutive days; suitably, during the course of treatment, both compounds will be administered within a specified period for at least 5 consecutive

days, followed by administration of Compound $A^2$ alone for at least 2 consecutive days - in this case, the duration of time will be at least 7 consecutive days; suitably, during the course of treatment, both compounds will be administered within a specified period for at least 5 consecutive days, followed by administration of Compound $A^2$ alone for at least 3 consecutive days - in this case, the duration of time will be at least 8 consecutive days; suitably, during the course of treatment, both compounds will be administered within a specified period for at least 5 consecutive days, followed by administration of Compound $A^2$ alone for at least 4 consecutive days - in this case, the duration of time will be at least 9 consecutive days; suitably, during the course of treatment, both compounds will be administered within a specified period for at least 5 consecutive days, followed by administration of Compound $A^2$ alone for at least 5 consecutive days - in this case, the duration of time will be at least 10 consecutive days; suitably, during the course of treatment, both compounds will be administered within a specified period for at least 7 consecutive days, followed by administration of Compound $A^2$ alone for at least 2 consecutive days - in this case, the duration of time will be at least 9 consecutive days; suitably, during the course of treatment, both compounds will be administered within a specified period for at least 14 consecutive days, followed by administration of Compound $A^2$ alone for at least 7 consecutive days - in this case, the duration of time will be at least 21 consecutive days; suitably, during the course of treatment, both compounds will be administered within a specified period for at least 30 consecutive days, followed by administration of Compound $A^2$ alone for at least 7 consecutive days - in this case, the duration of time will be at least 37 consecutive days. Suitably, during the course of treatment, both compounds will be administered within a specified period for from 1 to 3 consecutive days, followed by administration of Compound $A^2$ alone for from 3 to 7 consecutive days. Suitably, during the course of treatment, both compounds will be administered within a specified period for from 3 to 6 consecutive days, followed by administration of Compound $A^2$ alone for from 1 to 4 consecutive days. Suitably, during the course of treatment, both compounds will be administered within a specified period for 5 consecutive days, followed by administration of Compound $A^2$ alone for 2 consecutive days. Suitably, during the course of treatment, both compounds will be administered within a specified period for 2 consecutive days, followed by administration of Compound $A^2$ alone for from 3 to 7 consecutive days. Suitably, during the course of treatment, both compounds will be administered within a specified period for from 1 to 3 days over a 7 day period, and during the other days of the 7 day period Compound $A^2$ will be administered alone. Suitably, during the course of treatment, both compounds will be administered within a specified period for 2 days over a 7 day period, and during the other days of the 7 day period Compound $A^2$ will be administered alone.

Further regarding "specified period" administration:

Suitably, during the course of treatment, both compounds will be administered within a specified period for at least 1 day, followed by the administration of Compound $B^2$ alone for at least 1 day - in this case, the duration of time will be at least 2 days; suitably, during the course of treatment, both compounds will be administered within a specified period for at least 1 day, followed by administration of Compound $B^2$ alone for at least 2 days - in this case, the duration of time will be at least 3 days; suitably, during the course of treatment, both compounds will be administered within a specified period for at least 1 day, followed by administration of Compound $B^2$ alone for at least 3 days - in this case, the duration of time will be at least 4 days; suitably, during the course of treatment, both compounds will be administered within a specified period for at least 1 day, followed by administration of Compound $B^2$ alone for at least 4 days - in this case, the duration of time will be at least 5 days; suitably, during the course of treatment, both compounds will be administered within a specified period for at least 1 day, followed by administration of Compound $B^2$ alone for at least 5 days - in this case, the duration of time will be at least 6 days; suitably, during the course of treatment, both compounds will be administered within a specified period for at least 1 day, followed by administration of Compound $B^2$ alone for at least 6 days - in this case, the duration of time will be at least 7 days; suitably, during the course of treatment, both compounds will be administered within a specified period for at least 1 day, followed by administration of Compound $B^2$ alone for at least 7 days - in this case, the duration of time will be at least 8 days; suitably, during the course of treatment, both compounds will be administered within a specified period for at least 2 consecutive days, followed by administration of Compound $B^2$ alone for at least 1 day - in this case, the duration of time will be at least 3 days; suitably, during the course of treatment, both compounds will be administered within a specified period for at least 2 consecutive days, followed by administration of Compound $B^2$ alone for at least 2 consecutive days - in this case, the duration of time will be at least 4 days; suitably, during the course of treatment, both compounds will be administered within a specified period for at least 2 consecutive days, followed by administration of Compound $B^2$ alone for at least 3 consecutive days - in this case, the duration of time will be at least 5 days; suitably, during the course of treatment, both compounds will be administered within a specified period for at least 2 consecutive days, followed by administration of Compound $B^2$ alone for at least 4 consecutive days - in this case, the duration of time will be at least 6 days; suitably, during the course of treatment, both compounds will be administered within a specified period for at least 2 consecutive days, followed by administration of Compound $B^2$ alone for at least 5 consecutive days - in this case, the duration of time will be at least 7 days; suitably, during the course of treatment, both compounds will be administered within a specified period for at least 2 consecutive days, followed by administration of Compound $B^2$ alone for at least 6 consecutive days - in this case,

the duration of time will be at least 8 days; suitably, during the course of treatment, both compounds will be administered within a specified period for at least 2 consecutive days, followed by administration of Compound $B^2$ alone for at least 7 consecutive days - in this case, the duration of time will be at least 9 days; suitably, during the course of treatment, both compounds will be administered within a specified period for at least 3 consecutive days, followed by administration of Compound $B^2$ alone for at least 1 day - in this case, the duration of time will be at least 4 days; suitably, during the course of treatment, both compounds will be administered within a specified period for at least 3 consecutive days, followed by administration of Compound $B^2$ alone for at least 2 consecutive days - in this case, the duration of time will be at least 5 days; suitably, during the course of treatment, both compounds will be administered within a specified period for at least 3 consecutive days, followed by administration of Compound $B^2$ alone for at least 3 consecutive days - in this case, the duration of time will be at least 6 days; suitably, during the course of treatment, both compounds will be administered within a specified period for at least 3 consecutive days, followed by administration of Compound $B^2$ alone for at least 4 consecutive days - in this case, the duration of time will be at least 7 days; suitably, during the course of treatment, both compounds will be administered within a specified period for at least 3 consecutive days, followed by administration of Compound $B^2$ alone for at least 5 consecutive days - in this case, the duration of time will be at least 8 days; suitably, during the course of treatment, both compounds will be administered within a specified period for at least 3 consecutive days, followed by administration of Compound $B^2$ alone for at least 6 consecutive days - in this case, the duration of time will be at least 9 days; suitably, during the course of treatment, both compounds will be administered within a specified period for at least 3 consecutive days, followed by administration of Compound $B^2$ alone for at least 7 consecutive days - in this case, the duration of time will be at least 10 days; suitably, during the course of treatment, both compounds will be administered within a specified period for at least 4 consecutive days, followed by administration of Compound $B^2$ alone for at least 1 day - in this case, the duration of time will be at least 5 consecutive days; suitably, during the course of treatment, both compounds will be administered within a specified period for at least 4 consecutive days, followed by administration of Compound $B^2$ alone for at least 2 consecutive days - in this case, the duration of time will be at least 6 consecutive days; suitably, during the course of treatment, both compounds will be administered within a specified period for at least 4 consecutive days, followed by administration of Compound $B^2$ alone for at least 3 consecutive days - in this case, the duration of time will be at least 7 consecutive days; suitably, during the course of treatment, both compounds will be administered within a specified period for at least 4 consecutive days, followed by administration of Compound $B^2$ alone for at least 4 consecutive days - in this case, the duration of time will be at least 8 consecutive days; suitably, during the course of treatment, both compounds will be administered within a specified period for at least 4 consecutive days, followed by administration of Compound $B^2$ alone for at least 7 consecutive days - in this case, the duration of time will be at least 11 consecutive days; suitably, during the course of treatment, both compounds will be administered within a specified period for at least 5 consecutive days, followed by administration of Compound $B^2$ alone for at least 1 day - in this case, the duration of time will be at least 6 consecutive days; suitably, during the course of treatment, both compounds will be administered within a specified period for at least 5 consecutive days, followed by administration of Compound $B^2$ alone for at least 2 consecutive days - in this case, the duration of time will be at least 7 consecutive days; suitably, during the course of treatment, both compounds will be administered within a specified period for at least 5 consecutive days, followed by administration of Compound $B^2$ alone for at least 3 consecutive days - in this case, the duration of time will be at least 8 consecutive days; suitably, during the course of treatment, both compounds will be administered within a specified period for at least 5 consecutive days, followed by administration of Compound $B^2$ alone for at least 4 consecutive days - in this case, the duration of time will be at least 9 consecutive days; suitably, during the course of treatment, both compounds will be administered within a specified period for at least 5 consecutive days, followed by administration of Compound $B^2$ alone for at least 5 consecutive days - in this case, the duration of time will be at least 10 consecutive days; suitably, during the course of treatment, both compounds will be administered within a specified period for at least 7 consecutive days, followed by administration of Compound $B^2$ alone for at least 2 consecutive days - in this case, the duration of time will be at least 9 consecutive days; suitably, during the course of treatment, both compounds will be administered within a specified period for at least 14 consecutive days, followed by administration of Compound $B^2$ alone for at least 7 consecutive days - in this case, the duration of time will be at least 21 consecutive days; suitably, during the course of treatment, both compounds will be administered within a specified period for at least 30 consecutive days, followed by administration of Compound $B^2$ alone for at least 7 consecutive days - in this case, the duration of time will be at least 37 consecutive days. Suitably, during the course of treatment, both compounds will be administered within a specified period for from 1 to 3 consecutive days, followed by administration of Compound $B^2$ alone for from 3 to 7 consecutive days. Suitably, during the course of treatment, both compounds will be administered within a specified period for from 3 to 6 consecutive days, followed by administration of Compound $B^2$ alone for from 1 to 4 consecutive days. Suitably, during the course of treatment, both compounds will be administered within a specified period for 5 consecutive days, followed by administration of Compound $B^2$ alone for 2 consecutive days. Suitably, during the course of treatment, both compounds will be administered within a specified period for 2 consecutive days, followed by administration of Compound $B^2$ alone for from 3 to 7 consecutive days. Suitably, during the course of treatment, both compounds will be administered within a specified period for from

1 to 3 days over a 7 day period, and during the other days of the 7 day period Compound $B^2$ will be administered alone. Suitably, during the course of treatment, both compounds will be administered within a specified period for 2 days over a 7 day period, and during the other days of the 7 day period Compound $B^2$ will be administered alone.

Further regarding "specified period" administration:

Suitably, during the course of treatment, Compound $A^2$ and Compound $B^2$ will be administered within a specified period for from 1 to 3 days over a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for from 1 to 3 days over a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 3 days over a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 3 days over a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 2 days over a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 2 days over a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 1 day during a 7 day period, and during the other days of the 7 day period Compound $A^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for 1 day during a 7 day period, and during the other days of the 7 day period Compound $B^2$ will be administered alone. Suitably, this 7 day protocol is repeated for 2 cycles or for 14 days; suitably for 4 cycles or 28 days; suitably for continuous administration.

Suitably, during the course of treatment, Compound $A^2$ and Compound $B^2$ will be administered within a specified period for from 1 to 5 days over a 14 day period, and during the other days of the 14 day period Compound $A^2$ will be administered alone. Suitably, this 14 day protocol is repeated for 2 cycles or for 28 days; suitably for continuous administration.

Suitably, during the course of treatment, Compound $A^2$ and Compound $B^2$ will be administered within a specified period for from 1 to 5 days over a 14 day period, and during the other days of the 14 day period Compound $B^2$ will be administered alone. Suitably, this 14 day protocol is repeated for 2 cycles or for 28 days; suitably for continuous administration.

Suitably, if the compounds are not administered during a "specified period", they are administered sequentially. By the term "sequential administration", and derivates thereof, as used herein is meant that one of Compound $A^2$ and Compound $B^2$ is administered for 1 or more consecutive days and the other of Compound $A^2$ and Compound $B^2$ is subsequently administered for 1 or more consecutive days. Unless otherwise defined, the "sequential administration" and in all dosing protocols described herein, do not have to commence with the start of treatment and terminate with the end of treatment, it is only required that the administration of one of Compound $A^2$ and Compound $B^2$ followed by the administration of the other of Compound $A^2$ and Compound $B^2$, or the indicated dosing protocol, occur at some point during the course of treatment. Also, contemplated herein is a drug holiday utilized between the sequential administration of one of Compound $A^2$ and Compound $B^2$ and the other of Compound $A^2$ and Compound $B^2$. As used herein, a drug holiday is a period of days after the sequential administration of one of Compound $A^2$ and Compound $B^2$ and before the administration of the other of Compound $A^2$ and Compound $B^2$ where neither Compound $A^2$ nor Compound $B^2$ is administered. Suitably the drug holiday will be a period of days selected from: 1 day, 2 days, 3 days, 4 days, 5 days, 6

days, 7 days, 8 days, 9 days, 10 days, 11 days, 12 days, 13 days and 14 days.

Regarding sequential administration:

Suitably, one of Compound $A^2$ and Compound $B^2$ is administered for from 1 to 30 consecutive days, followed by an optional drug holiday, followed by administration of the other of Compound $A^2$ and Compound $B^2$ for from 1 to 30 consecutive days. Suitably, one of Compound $A^2$ and Compound $B^2$ is administered for from 1 to 21 consecutive days, followed by an optional drug holiday, followed by administration of the other of Compound $A^2$ and Compound $B^2$ for from 1 to 21 consecutive days. Suitably, one of Compound $A^2$ and Compound $B^2$ is administered for from 1 to 14 consecutive days, followed by a drug holiday of from 1 to 14 days, followed by administration of the other of Compound A and Compound $B^2$ for from 1 to 14 consecutive days. Suitably, one of Compound $A^2$ and Compound $B^2$ is administered for from 2 to 7 consecutive days, followed by a drug holiday of from 2 to 10 days, followed by administration of the other of Compound A and Compound $B^2$ for from 2 to 7 consecutive days.

Suitably, Compound $B^2$ will be administered first in the sequence, followed by an optional drug holiday, followed by administration of Compound $A^2$. Suitably, Compound $B^2$ is administered for from 1 to 21 consecutive days, followed by an optional drug holiday, followed by administration of Compound $A^2$ for from 1 to 21 consecutive days. Suitably, Compound $B^2$ is administered for from 3 to 21 consecutive days, followed by a drug holiday of from 1 to 14 days, followed by administration of Compound $A^2$ for from 3 to 21 consecutive days. Suitably, Compound $B^2$ is administered for from 3 to 21 consecutive days, followed by a drug holiday of from 3 to 14 days, followed by administration of Compound $A^2$ for from 3 to 21 consecutive days. Suitably, Compound $B^2$ is administered for 21 consecutive days, followed by an optional drug holiday, followed by administration of Compound $A^2$ for 14 consecutive days. Suitably, Compound $B^2$ is administered for 14 consecutive days, followed by a drug holiday of from 1 to 14 days, followed by administration of Compound $A^2$ for 14 consecutive days. Suitably, Compound $B^2$ is administered for 7 consecutive days, followed by a drug holiday of from 3 to 10 days, followed by administration of Compound $A^2$ for 7 consecutive days. Suitably, Compound $B^2$ is administered for 3 consecutive days, followed by a drug holiday of from 3 to 14 days, followed by administration of Compound $A^2$ for 7 consecutive days. Suitably, Compound $B^2$ is administered for 3 consecutive days, followed by a drug holiday of from 3 to 10 days, followed by administration of Compound $A^2$ for 3 consecutive days.

Suitably, Compound $A^2$ will be administered first in the sequence, followed by an optional drug holiday, followed by administration of Compound $B^2$. Suitably, Compound $A^2$ is administered for from 1 to 21 consecutive days, followed by an optional drug holiday, followed by administration of Compound $B^2$ for from 1 to 21 consecutive days. Suitably, Compound $A^2$ is administered for from 3 to 21 consecutive days, followed by a drug holiday of from 1 to 14 days, followed by administration of Compound $B^2$ for from 3 to 21 consecutive days. Suitably, Compound $A^2$ is administered for from 3 to 21 consecutive days, followed by a drug holiday of from 3 to 14 days, followed by administration of Compound $B^2$ for from 3 to 21 consecutive days. Suitably, Compound $A^2$ is administered for 21 consecutive days, followed by an optional drug holiday, followed by administration of Compound $B^2$ for 14 consecutive days. Suitably, Compound $A^2$ is administered for 14 consecutive days, followed by a drug holiday of from 1 to 14 days, followed by administration of Compound $B^2$ for 14 consecutive days. Suitably, Compound $A^2$ is administered for 7 consecutive days, followed by a drug holiday of from 3 to 10 days, followed by administration of Compound $B^2$ for 7 consecutive days. Suitably, Compound $A^2$ is administered for 3 consecutive days, followed by a drug holiday of from 3 to 14 days, followed by administration of Compound $B^2$ for 7 consecutive days. Suitably, Compound $A^2$ is administered for 3 consecutive days, followed by a drug holiday of from 3 to 10 days, followed by administration of Compound $B^2$ for 3 consecutive days. Suitably, Compound $A^2$ is administered for 7 consecutive days, followed by administration of Compound $B^2$ for 1 day. Suitably, Compound $A^2$ is administered for 6 consecutive days, followed by administration of Compound $B^2$ for 1 day. Suitably, Compound $B^2$ is administered for 1 day, followed by administration of Compound $A^2$ for 7 consecutive days. Suitably, Compound $B^2$ is administered for 1 day, followed by administration of Compound $A^2$ for 6 consecutive days.

It is understood that a "specified period" administration and a "sequential" administration can be followed by one or more cycles of repeat dosing or can be followed by an alternate dosing protocol, and a drug holiday may precede the repeat dosing or alternate dosing protocol.

Suitably, the amount of Compound $A^2$ administered as part of the combination according to the present invention will be an amount selected from about 250mg to about 1,500mg; suitably, the amount will be selected from about 500mg to about 1,250mg; suitably, the amount will be selected from about 750mg to about 1,250mg; suitably, the amount will be selected from about 1,000mg to about 1,250mg; suitably, the amount will be 250mg, suitably, the amount will be 500mg, suitably, the amount will be 750mg, suitably, the amount will be 1,000mg, suitably, the amount will be 1,250mg; suitably, the amount will be 1,500mg. Accordingly, the amount of Compound $A^2$ administered as part of the combination according to the present invention will be an amount selected from about 250mg to about 1,500 mg. For example, the amount of Compound $A^2$ administered as part of the combination according to the present invention is suitably selected from 250mg, 500mg, 750mg, 1,000mg, 1,250mg and 1,500mg. Suitably, the selected amount of Compound $A^2$ is administered from 1 to 4 times a day, in one or more tablets. Suitably, the selected amount of Compound $A^2$ is administered

twice a day, in one or more tablets. Suitably, the selected amount of Compound $A^2$ is administered once a day, in one or more tablets. Suitably, the administration of Compound $A^2$ will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

Suitably, the amount of Compound $B^2$ administered as part of the combination according to the present invention will be an amount selected from about 5mg to about 500mg; suitably, the amount will be selected from about 25mg to about 400mg; suitably, the amount will be selected from about 30mg to about 375mg; suitably, the amount will be selected from about 35mg to about 350mg; suitably, the amount will be selected from about 40mg to about 300mg; suitably, the amount will be selected from about 45mg to about 275mg; suitably, the amount will be selected from about 50mg to about 250mg; suitably, the amount will be selected from about 55mg to about 225mg; suitably, the amount will be selected from about 60mg to about 200mg; suitably, the amount will be selected from about 65mg to about 175mg; suitably, the amount will be selected from about 70mg to about 150mg; suitably, the amount will be selected from about 50mg to about 300mg; suitably, the amount will be selected from about 75mg to about 150mg; suitably, the amount will be about 100mg. Accordingly, the amount of Compound $B^2$ administered as part of the combination according to the present invention will be an amount selected from about 5mg to about 500mg. For example, the amount of Compound $B^2$ administered as part of the combination according to the present invention can be 5mg, 10mg, 15mg, 20mg, 25mg, 30mg, 35mg, 40mg, 45mg, 50mg, 55mg, 60mg, 65mg, 70mg, 75mg, 80mg, 85mg, 90mg, 95mg, 100mg, 105mg, 110mg, 115mg, 120mg, 125mg, 130mg, 135mg, 140mg, 145mg, 150mg, 175mg, 200mg, 225mg, 250mg, 275mg, 300mg, 325mg, 350mg, 375mg, 400mg, 425mg, 450mg, 475mg or 500mg. Suitably, the selected amount of Compound $B^2$ is administered twice a day. Suitably, the selected amount of Compound $B^2$ is administered once a day. Suitably, the administration of Compound $B^2$ will begin as a loading dose. Suitably, the loading dose will be an amount from 2 to 100 times the maintenance dose; suitably from 2 to 10 times; suitably from 2 to 5 times; suitably 2 times; suitably 3 times; suitably 4 times; suitably 5 times. Suitably, the loading does will be administered from 1 to 7 days; suitably from 1 to 5 days; suitably from 1 to 3 days; suitably for 1 day; suitably for 2 days; suitably for 3 days, followed by a maintenance dosing protocol.

As used herein, all amounts specified for Compound $A^2$ and Compound $B^2$ are indicated as the administered amount of free or unsalted and unsolvated compound per dose.

The method of the present disclosure may also be employed with other therapeutic methods of cancer treatment.

While it is possible that, for use in therapy, therapeutically effective amounts of the combinations of the present invention may be administered as the raw chemical, it is preferable to present the combinations as a pharmaceutical composition or compositions. Accordingly, the invention further provides pharmaceutical compositions, which include Compound $A^2$ and/or Compound $B^2$, and one or more pharmaceutically acceptable carriers. The combinations of the present invention are as described above. The carrier(s) must be acceptable in the sense of being compatible with the other ingredients of the formulation, capable of pharmaceutical formulation, and not deleterious to the recipient thereof. In accordance with another aspect of the invention there is also provided a process for the preparation of a pharmaceutical formulation including admixing Compound $A^2$ and/or Compound $B^2$ with one or more pharmaceutically acceptable carriers. As indicated above, such elements of the pharmaceutical combination utilized may be presented in separate pharmaceutical compositions or formulated together in one pharmaceutical formulation.

Pharmaceutical formulations may be presented in unit dose forms containing a predetermined amount of active ingredient per unit dose. As is known to those skilled in the art, the amount of active ingredient per dose will depend on the condition being treated, the route of administration and the age, weight and condition of the patient. Preferred unit dosage formulations are those containing a daily dose or sub-dose, or an appropriate fraction thereof, of an active ingredient. Furthermore, such pharmaceutical formulations may be prepared by any of the methods well known in the pharmacy art.

Compound $A^2$ and Compound $B^2$ may be administered by any appropriate route. Suitable routes include oral, rectal, nasal, topical (including buccal and sublingual), vaginal, and parenteral (including subcutaneous, intramuscular, intravenous, intradermal, intrathecal, and epidural). It will be appreciated that the preferred route may vary with, for example, the condition of the recipient of the combination and the cancer to be treated. It will also be appreciated that each of the agents administered may be administered by the same or different routes and that Compound $A^2$ and Compound $B^2$ may be compounded together in a pharmaceutical composition/formulation. Suitably, Compound $A^2$ and Compound $B^2$ are administered in separate pharmaceutical compositions.

The compounds or combinations of the current invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier may include a prolonged release material, such as glyceryl monostearate or glyceryl distearate, alone or with a wax. The

amount of solid carrier varies widely but, suitably, may be from about 25 mg to about 1 g per dosage unit. When a liquid carrier is used, the preparation will suitably be in the form of a syrup, elixir, emulsion, soft gelatin capsule, sterile injectable liquid such as an ampoule, or an aqueous or nonaqueous liquid suspension.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

It should be understood that in addition to the ingredients mentioned above, the formulations may include other agents conventional in the art having regard to the type of formulation in question, for example those suitable for oral administration may include flavoring agents.

As indicated, therapeutically effective amounts of the combinations of the invention (Compound $A^2$ in combination with Compound $B^2$) are administered to a human. Typically, the therapeutically effective amount of the administered agents of the present invention will depend upon a number of factors including, for example, the age and weight of the subject, the precise condition requiring treatment, the severity of the condition, the nature of the formulation, and the route of administration. Ultimately, the therapeutically effective amount will be at the discretion of the attending physician.

The combinations of the present invention are tested for efficacy, advantageous and synergistic properties according to known procedures.

Suitably, the combinations of the invention are tested for efficacy, advantageous and synergistic properties generally according to the following combination cell proliferation and apoptosis assays.

**In vitro cell growth inhibition by Compound A, Compound B and their combination in tumor cell lines**

**Methods:**

**Cell lines and growth conditions**

Human breast tumor cell lines BT474, HCC1419, HCC1954, JIMT1, MDA-MB-361, SK-BR-3 and UACC893 were cultured in RPMI 1640 medium containing 10 % FBS. BT-474-J4, a breast cancer cell line acquired resistant to Compound A was cultured in RPMI 1640 medium containing 10% FBS and 1 μM of Compound A. KPL4 breast cancer line was cultured in DMEM containing 5% FBS. All lines were kept in a humidified incubator at 37°C in 95% air and 5% $CO_2$. JIMT-1 is a cell line established from a tumor of a patient with trastuzumab-resistant breast cancer. BT-474-J4 is a single cell clone derived from a culture of BT-474 cells that were selected to grow in Compound A to a concentration of 3 μM.

**Cell growth inhibition assay and combination data analysis.**

All cells were cultured for a minimum of 72 hours prior to cell plating. Cells were assayed in a 96-well tissue culture plate (NUNC 136102) of RPMI medium containing 10 % FBS for all cells at 2,000 cells per well except KPL4, which were plated in DMEM containing 5% FBS at 500 cells per well. Approximately 24 hours after plating, cells were exposed to ten, two-fold or three-fold serial dilutions of compound or the combination of the two agents at a constant molar to molar ratio of 1:1 Compound A to Compound B in RPMI media containing 10% FBS or DMEM containing 5% FBS for KPL4. Cells were incubated in the presence of compounds for 3 days. ATP levels were determined by adding Cell Titer Glo® (Promega) according to the manufacturer's protocol. Briefly, Cell Titer Glo® was added to each plate, incubated for 20 minutes then the luminescent signal was read on a SpectraMax L plate reader with a 0.5 sec integration time.

Inhibition of cell growth was estimated after treatment with compound or a combination of compounds for three days and comparing the signal to cells treated with vehicle (DMSO). Cell growth was calculated relative to vehicle (DMSO) treated control wells. Concentration of compound that inhibits 50% of control cell growth ($IC_{50}$) was interpolated using nonlinear regression with the equation, y=(A+(B-A)/(1+(C/x)^D)))), where A is the minimum response ($y_{min}$), B is the maximum response ($y_{max}$), C is the inflection point of the curve ($EC_{50}$) and D is the Hill coefficient.

Combination effects on potency were evaluated using Combination Index (CI) which was calculated with the back-interpolated $IC_{50}$ values and the mutually non-exclusive equation derived by Chou and Talalay (Chou TC, Talalay P. Quantitative analysis of dose-effect relationships: the combined effects of multiple drugs or enzyme inhibitors. Adv Enzyme Regul 1984;22:27-55):

$$CI = Da/IC_{50}(a) + Db/IC_{50}(b) + (Da \times Db)/(IC_{50}(a) \times IC_{50}(b))$$

where $IC_{50}(a)$ is the $IC_{50}$ of the inhibitor A; $IC_{50}(b)$ is the $IC_{50}$ for the inhibitor B; Da is the concentration of the inhibitor A in combination with the inhibitor B that inhibited 50 % of cell growth; and Db is the concentration of inhibitor B in combination with the inhibitor A that inhibited 50% of cell growth. In general, a CI value <0.9, between 0.9 and 1.1, or >1.1 indicates synergy, additivity and antagonism, respectively. In general, the smaller the CI number, the greater is the strength of synergy.

The combination effects on the response scale were quantified by Excess Over Highest Single Agent (EOHSA) [Liu L, Shi H, Liu Y, Anderson A, Peterson J, Greger J, Martin AM, Gilmer TM. Synergistic Effects of Foretinib with HER-Targeted Agents in MET and HER1- or HER2-Coactivated Tumor Cells. Mol Cancer Ther. 2011;10:518-30]. EOHSA values were defined as increases in improvement (here, in 'percentage points' (ppts) difference) produced by the combination over the best single agent at its component dose level for the combination. For single agent and combination treatments, cells were exposed to compounds at a fixed-dose-ratio, and dose response curves were fit to the experimental data and analyzed using regression models described above. At specified total dose levels of $IC_{50}$ along the dose response curve, the corresponding component dose levels for each single agent were calculated. EOHSA was determined by the difference between the response value from the combination (50% inhibition) and the greater response value from the two single agents at the corresponding component dose levels.

**Cell apoptosis induction- caspase-3/7 activation.**

For investigation of the induction of apoptosis, cells were plated at 5,000 cells per well in a 96-well tissue culture plate and allowed to attach for approximately 24 hours. Cells were exposed to ten, two-fold or three-fold serial dilutions of compound or the combination of the two agents at a constant molar to molar ratio of 10:1 Compound A to Compound B in RPMI media containing 10% FBS or DMEM containing 5% FBS for KPL4. Twenty-four hours after compound treatment, caspase 3/7 activity was determined with the Caspase Glo™ 3/7 (Promega, cat G8093) according to the instructions provided by the manufacturer.

Figure 1. Dose response curves of cell growth inhibition. Cell growth was determined by , Cell Titer Glo® assay as described in the method 72 hours post treatment of BT474, KPL-4 and BT474-J4 cells with Compound A, Compound B or the 1 to 1 molar ratio combination of Compound A and Compound B.

Figure 2. Dose response curves of apoptosis induction. Apoptosis was determined by caspase 3/7 activation assay as described in the method 24 hours post treatment of BT474, KPL-4 and BT474-J4 cells with Compound A, Compound B or the 10 to 1 molar ratio combination of Compound A and Compound B.

**Results:**

As used in the specification and the "results section", Compound A is N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof; Compound B is *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt or solvate thereof.

**Cell growth inhibition by Compound A, Compound B and the combination of Compound A with Compound B.**

The effects of cell growth inhibition by Compound A, Compound B and the combination of Compound A and Compound B were determined in nine HER2 positive human breast tumor lines. The mean $IC_{50}$s (from at least two independent experiments) and the combination effects at $IC_{50}$s are summarized in Table 1. Representative dose response curves for BT474, KPL-4 and BT474-J4 cell lines are provided in Figure 1.

The combination of Compound A and Compound B was nearly additive with CI values from 0.89 to 1.04 in HCC1419, SK-BR-3 and BT474 lines, which were highly sensitive to Compound A ($IC_{50} \leq 0.134$ μM), however they were insensitive to Compound B ($IC_{50} \geq 2.6$ μM) as a single agent. In contrast, the combination of Compound A and Compound B was synergistic (CI= 0.25-0.75) and enhanced the inhibition of cell growth (EOHSA = 11-31 ppts) in KPL-4, UACC893 and HCC1954 lines, all of which were moderately sensitive to Compound A ($IC_{50}$s = 0.9-1.7 μM) and displayed sensitivity to Compound B with $IC_{50}$ values between 0.5 μM and 3.7 μM as a single agent. Tumor cell lines BT474-J4, and MDA-MB-361 were resistant to Compound A ($IC_{50} \geq 9.2$ μM), however sensitive to Compound B with $IC_{50}$ values of 0.5 and 0.7 μM respectively. The combination of Compound A and Compound B was synergistic (CI =0.4)

in BT474-J4 line, and enhanced the inhibition of cell growth in both BT474-J4 and MDA-MB-361 lines (EOHSA = 17 and 20 ppts respectively). JIMT-1, an HER2+ breast cancer line established from a tumor of a patient trastuzumab-resistant breast cancer. (Tanner et al, Mol Cancer Ther 2004;3:1585-92), is resistant to both single agents Compound A and Compound B ($IC_{50}$ ≥6.8 μM). Enhanced cell growth inhibition was observed with this combination in JIMT-1 cells (EOSHA = 12 ppts); however, the benefit was observed at relatively high concentrations.

**Apoptosis induction by Compound A, Compound B and the combination of Compound A with Compound B.**

A subset of HER2+ breast cell lines, BT474, KPL-4 and BT474-J4 were further evaluated for the ability of Compound A, Compound B or the combination of Compound A and Compound B to induce apoptosis. Activation of caspase 3 or 7 is a hallmark of induction of apoptosis. Representative caspase 3/7 activation curves for BT474, KPL-4 and BT474-J4 cells are provided in Figure 2. Compound A alone increased caspase 3/7 activity in a dose dependent manner in the BT474 line. A slight increase in caspase 3/7 activity was observed with Compound A alone at 10 μM in KPL4 and BT474-J4 lines. Compound B alone slightly increased caspase 3/7 activity at 1 μM in BT474 and BT474-J4 lines, but not in the KPL-4 line. However the combination of Compound A and Compound B enhanced the levels of observed caspase 3/7 activity in all three cell lines.

Table 1. Cell growth inhibition by Compound A, Compound B and their combination in tumor cell lines.

| Human breast cancer cell lines | Single Agent $IC_{50}$ (μM), mean±STD | | Combination $IC_{50}$ (μM), mean±STD | Combination effects at $IC_{50}$ | |
|---|---|---|---|---|---|
| | Compound A | Compound B | Compound A or Compound B | CI, mean±STD | EOHSA (ppts), mean±STD |
| HCC1419 | 0.077 ± 0.017 | 2.623 ± 0.548 | 0.075 ± 0.010 | 1.04 ± 0.10 | 0.4 ± 1.7 |
| SK-BR-3 | 0.105 ± 0.039 | 4.189 ± 1.583 | 0.088 ± 0.025 | 0.89 ± 0.08 | 2.3 ± 0.7 |
| BT-474* | 0.134 ± 0.049 | 3.301 ± 1.102 | 0.120 ± 0.035 | 0.98 ± 0.02 | 1.0 ± 0.7 |
| KPL4* | 0.860 ± 0.039 | 0.113 ± 0.012 | 0.069 ± 0.003 | 0.75 ± 0.05 | 11.2 ± 0.8 |
| UACC893* | 1.122 ± 0.150 | 0.505 ± 0.303 | 0.135 ± 0.037 | 0.45 ± 0.10 | 31.3 ± 3.1 |
| HCC1954* | 1.725 ± 0.165 | 3.665 ± 2.971 | 0.237 ± 0.014 | 0.25 ± 0.09 | 27.8 ± 4.7 |
| BT-474-J4* | 9.229 ± 3.825 | 0.670 ± 0.026 | 0.238 ± 0.033 | 0.40 ± 0.09 | 17.4 ± 2.3 |
| MDA-MB-361* | >10 | 0.758 ± 0.074 | 0.474 ± 0.041 | N/A | 11.6 ± 0.3 |
| JIMT-1* | >10 | 6.809 ± 2.131 | 2.125 ± 0.065 | N/A | 20.4 ± 1.7 |
| *Cell lines with PIK3CA mutations. N/A: not applicable; CI:Combination index, non-mutually exclusive; ppts: percentage points | | | | | |

Because the combinations of the present invention are active in the above assays they exhibit advantageous therapeutic utility in treating cancer.

Suitably, the present invention relates to a combination for use in a method for treating or lessening the severity of a cancer selected from: brain (gliomas), glioblastomas, Bannayan-Zonana syndrome, Cowden disease, Lhermitte-Duclos disease, breast, inflammatory breast cancer, Wilm's tumor, Ewing's sarcoma, Rhabdomyosarcoma, ependymoma, medulloblastoma, colon, head and neck, kidney, lung, liver, melanoma, ovarian, pancreatic, prostate, sarcoma, osteosarcoma, giant cell tumor of bone, thyroid,

Lymphoblastic T cell leukemia, Chronic myelogenous leukemia, Chronic lymphocytic leukemia, Hairy-cell leukemia, acute lymphoblastic leukemia, acute myelogenous leukemia, Chronic neutrophilic leukemia, Acute lymphoblastic T cell leukemia, Plasmacytoma, Immunoblastic large cell leukemia, Mantle cell leukemia, Multiple myeloma Megakaryoblastic leukemia, multiple myeloma, acute megakaryocytic leukemia, promyelocytic leukemia, Erythroleukemia, malignant lymphoma, hodgkins lymphoma, non-hodgkins lymphoma, lymphoblastic T cell lymphoma, Burkitt's lymphoma, follicular lymphoma,

neuroblastoma, bladder cancer, urothelial cancer, lung cancer, vulval cancer, cervical cancer, endometrial cancer, renal cancer, mesothelioma, esophageal cancer, salivary gland cancer, hepatocellular cancer, gastric cancer, nasopharangeal cancer, buccal cancer, cancer of the mouth, GIST (gastrointestinal stromal tumor) and testicular cancer.

Suitably, the present invention relates to a combination for use in a method for treating or lessening the severity of a cancer selected from: brain (gliomas), glioblastomas, Bannayan-Zonana syndrome, Cowden disease, Lhermitte-Duclos disease, breast, colon, head and neck, kidney, lung, liver, melanoma, ovarian, pancreatic, prostate, sarcoma and thyroid.

Suitably, the present invention relates to a combination for use in a method for treating or lessening the severity of a cancer selected from ovarian, breast, pancreatic and prostate.

Suitably, the present invention relates to a combination for use in a method of treating or lessening the severity of a cancer that is either wild type or mutant for Ras/Raf and either wild type or mutant for PIK3CA/PTEN. This includes patients who are wild type for both Ras/Raf and PIK3CA/PTEN, mutant for both Ras/Raf and PIK3CA/PTEN, mutant for Ras/Raf and wild type for PIK3CA/PTEN and wild type for Ras/Raf and mutant for PIK3CA/PTEN. The present invention also relates to a combination for use in a method of treating or lessening the severity of a cancer that has activated AKT, e.g., by mutation or amplification of AKT1, AKT2 or AKT3 genes. The present invention also relates to a combination for use in a method of treating or lessening the severity of a cancer that has activated EGFR or ErbB-2, e.g., by mutation, amplification of the gene or overexpression of the protein.

The term "wild type" as is understood in the art refers to a polypeptide or polynucleotide sequence that occurs in a native population without genetic modification. As is also understood in the art, a "mutant" includes a polypeptide or polynucleotide sequence having at least one modification to an amino acid or nucleic acid compared to the corresponding amino acid or nucleic acid found in a wild type polypeptide or polynucleotide, respectively. Included in the term mutant is Single Nucleotide Polymorphism (SNP) where a single base pair distinction exists in the sequence of a nucleic acid strand compared to the most prevalently found (wild type) nucleic acid strand.

Cancers that are either wild type or mutant for Ras/Raf, PIK3CA/PTEN, AKT, EGFR or ErbB-2 or have amplification of PIK3CA, AKT, EGFR or ErbB-2 genes or have overexpression of EGFR or ErbB2 protein are identified by known methods.

For example, wild type or mutant Ras/Raf, PIK3CA/PTEN, AKT EGFR or ErbB-2 tumor cells can be identified by DNA amplification and sequencing techniques, DNA and RNA detection techniques, including, but not limited to Northern and Southern blot, respectively, and/or various biochip and array technologies or in-situ hybridization. Wild type and mutant polypeptides can be detected by a variety of techniques including, but not limited to immunodiagnostic techniques such as ELISA, Western blot or immunocytochemistry.

This invention provides a combination comprising N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof.

This invention also provides for a combination comprising N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, for use in therapy.

This invention also provides for a combination comprising N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, for use in treating cancer.

This invention also provides a pharmaceutical composition comprising a combination of N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof.

This invention also provides a combination kit comprising N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate

and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof.

This invention also provides for the use of a combination comprising N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, in the manufacture of a medicament.

This invention also provides for the use of a combination comprising N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, thereof, in the manufacture of a medicament to treat cancer.

This invention also provides a combination of, or a combination kit comprising such a combination, N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, suitably the ditosylate monohydrate salt, thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt, suitably the hydrochloride salt, and their uses, as described in the claims.

## Experimental Details

### Example 1 - Capsule Composition

An oral dosage form for administering a combination of the present invention is produced by filing a standard two piece hard gelatin capsule with the ingredients in the proportions shown in Table I, below.

Table I

| INGREDIENTS | AMOUNTS |
|---|---|
| N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate (the ditosylate monohydrate salt of Compound A) | 250mg |
| *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride (the hydrochloride salt of Compound B) | 75mg |
| Mannitol | 250 mg |
| Talc | 125 mg |
| Magnesium Stearate | 8 mg |

### Example 2 - Capsule Composition

An oral dosage form for administering one of the compounds of the present invention is produced by filing a standard two piece hard gelatin capsule with the ingredients in the proportions shown in Table II, below.

Table II

| INGREDIENTS | AMOUNTS |
|---|---|
| N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate (the ditosylate monohydrate salt of Compound A) | 250mg |
| Mannitol | 55 mg |
| Talc | 16 mg |
| Magnesium Stearate | 4 mg |

### Example 3 - Capsule Composition

An oral dosage form for administering one of the compounds of the present invention is produced by filing a standard two piece hard gelatin capsule with the ingredients in the proportions shown in Table III, below.

Table III

| INGREDIENTS | AMOUNTS |
|---|---|
| *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride (the hydrochloride salt of Compound B) | 75mg |
| Mannitol | 250mg |
| Talc | 125mg |
| Magnesium Stearate | 8mg |

Example 4 - Tablet Composition

The sucrose, microcrystalline cellulose and the compounds of the invented combination, as shown in Table IV below, are mixed and granulated in the proportions shown with a 10% gelatin solution. The wet granules are screened, dried, mixed with the starch, talc and stearic acid, then screened and compressed into a tablet.

Table IV

| INGREDIENTS | AMOUNTS |
|---|---|
| N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate (the ditosylate monohydrate salt of Compound A) | 250mg |
| *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride (the hydrochloride salt of Compound B) | 75mg |
| Microcrystalline cellulose | 300mg |
| sucrose | 10mg |
| starch | 40mg |
| talc | 20mg |
| stearic acid | 5mg |

Example 5 - Tablet Composition

The sucrose, microcrystalline cellulose and one of the compounds of the invented combination, as shown in Table V below, are mixed and granulated in the proportions shown with a 10% gelatin solution. The wet granules are screened, dried, mixed with the starch, talc and stearic acid, then screened and compressed into a tablet.

Table V

| INGREDIENTS | AMOUNTS |
|---|---|
| N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate (the ditosylate monohydrate salt of Compound A) | 250mg |
| Microcrystalline cellulose | 30mg |
| sucrose | 4mg |
| starch | 2mg |
| talc | 1mg |
| stearic acid | 0.5mg |

Example 6 - Tablet Composition

The sucrose, microcrystalline cellulose and one of the compounds of the invented combination, as shown in Table VI below, are mixed and granulated in the proportions shown with a 10% gelatin solution. The wet granules are screened, dried, mixed with the starch, talc and stearic acid, then screened and compressed into a tablet.

Table VI

| INGREDIENTS | AMOUNTS |
|---|---|
| *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride (the hydrochloride salt of Compound B) | 75mg |
| Microcrystalline cellulose | 300mg |

(continued)

| INGREDIENTS | AMOUNTS |
|---|---|
| sucrose | 40mg |
| starch | 20mg |
| talc | 10mg |
| stearic acid | 5mg |

## Claims

1. A combination comprising:

(i) a compound of Structure (I):

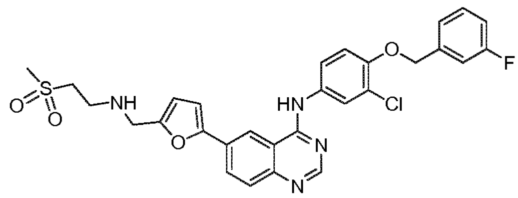

(I);

or a pharmaceutically acceptable hydrate and/or salt thereof; and
(ii) a compound of Structure (II):

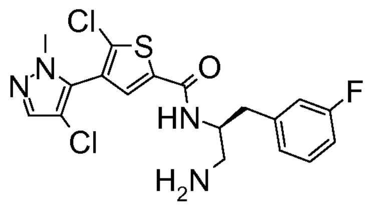

(II)

or a pharmaceutically acceptable salt thereof.

2. A combination according to claim 1 where the compound of Structure (I) is in the form of a ditosylate monohydrate salt and the compound of Structure (II) is in the form of a hydrochloride salt.

3. A combination kit comprising a combination according to claim 1 or claim 2 together with a pharmaceutically acceptable carrier or carriers.

4. A combination according to claim 1 or claim 2 or a combination kit according to claim 3 where the amount of the compound of Structure (I) is an amount selected from 750mg to 1,250mg, and the amount of the compound of Structure (II) is an amount selected from 50mg to 300mg.

5. Use of a combination according to claim 1 or claim 2 or of a combination kit according to claim 3 or of a combination or combination kit according to claim 4 in the manufacture of a medicament or medicaments for the treatment of cancer.

6. A combination or combination kit as claimed in any one of claims 1 to 4 for use in the treatment of cancer.

7. A combination or combination kit for use in the treatment of cancer, comprising a therapeutically effective amount of a combination of N-{3-Chloro-4-[(3-fluorobenzyl)oxy]phenyl}-6-[5-({[2-(methanesulphonyl)ethyl]amino}methyl)-2-furyl]-4-quinazolinamine, or a pharmaceutically acceptable hydrate and/or salt thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide, or a pharmaceutically acceptable salt thereof,
wherein the combination is administered within a specified period, and
wherein the combination is administered for a duration of time.

**8.** A combination or combination kit for use according to claim 7 wherein the amount of N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate is selected from about 750mg to about 1,250mg, and that amount is administered once per day in one or more tablets, and the amount of *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride, is selected from about 50mg to about 300mg, and that amount is administered once per day.

**9.** A combination or combination kit for use according to claim 8 wherein N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride, are administered within 12 hours of each other for from 1 to 3 consecutive days followed by administration of N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate for from 3 to 7 consecutive days, optionally followed by one or more cycles of repeat dosing.

**10.** A combination or combination kit for use according to claim 8 wherein N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride, are administered for at least 7 consecutive days.

**11.** A combination or combination kit for use according to claim 7 wherein N-{3-Chloro-4-[(3-fluorobenzyl)oxy]phenyl}-6-[5-({[2-(methanesulphonyl)ethyl]amino}methyl)-2-furyl]-4-quinazolinamine or a pharmaceutically acceptable hydrate and/or salt thereof, and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide or a pharmaceutically acceptable salt thereof, are administered within 12 hours of each other for at least 5 consecutive days.

**12.** A combination or combination kit for use according to claim 10 wherein N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate and *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride, are administered for at least 14 consecutive days.

**13.** A combination or combination kit for use according to claim 7 or claim 8 wherein the compound N-{3-Chloro-4-[(3-fluorobenzyl)oxy]phenyl}-6-[5-({[2-(methanesulphonyl)ethyl]amino}methyl)-2-furyl]-4-quinazolinamine ditosylate monohydrate is first administered in a loading dose for from 1 to 3 days followed by maintenance dose administration of the compound, or the compound *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide hydrochloride is first administered in a loading dose for from 1 to 3 days followed by maintenance dose administration of the compound.

**14.** A use according to claim 5, or a combination or combination kit for use according to any one of claims 4 and 6 to 13, wherein the cancer is either wild type or mutant for Ras/Raf, PIK3CA/PTEN, AKT, EGFR or ErbB-2 or has amplification of PIK3CA, AKT, EGFR or ErbB-2 genes or has overexpression of EGFR or ErbB2 protein.

**15.** A use according to claim 5, or a combination or combination kit for use according to any one of claims 4 and 6 to 13, wherein the cancer is selected from ovarian, breast, pancreatic and prostate.

**Patentansprüche**

**1.** Kombination, umfassend:

(i) eine Verbindung der Struktur (I):

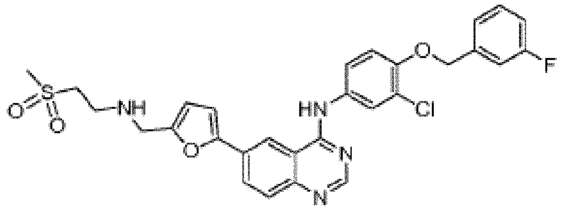

(I);

oder ein pharmazeutisch unbedenkliches Hydrat und/oder Salz davon; und
(ii) eine Verbindung der Struktur (II):

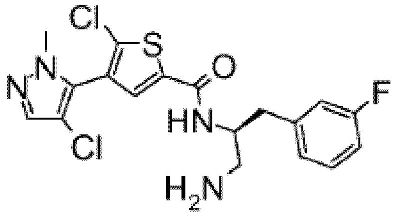

(II)

oder ein pharmazeutisch unbedenkliches Salz davon.

2. Kombination nach Anspruch 1, wobei die Verbindung der Struktur (I) in Form eines Ditosylat-Monohydrat-Salzes vorliegt und die Verbindung der Struktur (II) in Form eines Hydrochlorid-Salzes vorliegt.

3. Kombinationskit, umfassend eine Kombination nach Anspruch 1 oder Anspruch 2 gemeinsam mit (einem) pharmazeutisch unbedenklichen Träger bzw. Trägern.

4. Kombination nach Anspruch 1 oder Anspruch 2 oder Kombinationskit nach Anspruch 3, wobei die Menge an Verbindung der Struktur (I) eine Menge, ausgewählt von 750 mg bis 1 250 mg, ist und die Menge an Verbindung der Struktur (II) eine Menge, ausgewählt aus 50 mg bis 300 mg, ist.

5. Verwendung einer Kombination nach Anspruch 1 oder Anspruch 2 oder eines Kombinationskits nach Anspruch 3 oder einer Kombination oder eines Kombinationskits nach Anspruch 4 in der Herstellung eines Arzneimittels bzw. von Arzneimitteln für die Behandlung von Krebs.

6. Kombination oder Kombinationskit nach einem der Ansprüche 1 bis 4, zur Verwendung in der Behandlung von Krebs.

7. Kombination oder Kombinationskit zur Verwendung in der Behandlung von Krebs, umfassend eine therapeutisch wirksame Menge einer Kombination von N-{3-Chlor-4-[(3-fluorbenzyl)oxy]phenyl}-6-[5-({[2-(methansulfonyl)ethyl]amino}methyl)-2-furyl]-4-chinazolinamin, oder einem pharmazeutisch unbedenklichen Hydrat und/oder Salz davon, und *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid, oder einem pharmazeutisch unbedenklichen Salz davon,
wobei die Kombination innerhalb eines bestimmten Zeitraums verabreicht wird, und
wobei die Kombination für eine Zeitdauer verabreicht wird.

8. Kombination oder Kombinationskit zur Verwendung nach Anspruch 7, wobei die Menge an N-{3-Chlor-4-[(3-fluorbenzyl)oxy]phenyl}-6-[5-({[2-(methansulfonyl)ethyl]amino}methyl)-2-furyl]4-chinazolinamin-Ditosylat-Monohydrat aus ungefähr 750 mg bis ungefähr 1 250 mg ausgewählt ist und diese Menge einmal pro Tag in einer oder mehreren Tabletten verabreicht wird, und die Menge an *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid-Hydrochlorid aus ungefähr 50 mg bis ungefähr 300 mg ausgewählt ist und diese Menge einmal pro Tag verabreicht wird.

9. Kombination oder Kombinationskit zur Verwendung nach Anspruch 8, wobei N-{3-Chlor-4-[(3-fluorbenzyl)oxy]phenyl}-6-[5-({[2-(methansulfonyl)ethyl]amino}methyl)-2-furyl]4-chinazolinamin-Ditosylat-Monohydrat und *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid-Hydrochlorid innerhalb von 12 Stunden voneinander über 1 bis 3 aufeinanderfolgende Tage verabreicht werden, wonach

sich die Verabreichung von N-{3-Chlor-4-[(3-fluorbenzyl)oxy]phenyl}-6-[5-({[2-(methansulfonyl)ethyl]amino}methyl)-2-furyl]4-chinazolinamin-Ditosylat-Monohydrat 3 bis 7 aufeinanderfolgende Tage lang anschließt, gegebenenfalls gefolgt von einem oder mehreren Wiederholungsdosierungszyklen.

**10.** Kombination oder Kombinationskit zur Verwendung nach Anspruch 8, wobei N-{3-Chlor-4-[(3-fluorbenzyl)oxy]phenyl}-6-[5-({[2-(methansulfonyl)ethyl]amino}methyl)-2-furyl]4-chinazolinamin-Ditosylat-Monohydrat und *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid-Hydrochlorid über mindestens 7 aufeinanderfolgende Tage verabreicht werden.

**11.** Kombination oder Kombinationskit zur Verwendung nach Anspruch 7, wobei N-{3-Chlor-4-[(3-fluorbenzyl)oxy]phenyl}-6-[5-({[2-(methansulfonyl)ethyl]amino}methyl)-2-furyl]4-chinazolinamin oder ein pharmazeutisch unbedenkliches Hydrat und/oder Salz davon und *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid oder ein pharmazeutisch unbedenkliches Salz davon innerhalb von 12 Stunden voneinander über mindestens 5 aufeinanderfolgende Tage verabreicht werden.

**12.** Kombination oder Kombinationskit zur Verwendung nach Anspruch 10, wobei N-{3-Chlor-4-[(3-fluorbenzyl)oxy]phenyl}-6-[5-({[2-(methansulfonyl)ethyl]amino}methyl)-2-furyl]4-chinazolinamin-Ditosylat-Monohydrat und *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid-Hydrochlorid über mindestens 14 aufeinanderfolgende Tage verabreicht werden.

**13.** Kombination oder Kombinationskit zur Verwendung nach Anspruch 7 oder Anspruch 8, wobei die Verbindung N-{3-Chlor-4-[(3-fluorbenzyl)oxy]phenyl}-6-[5-({[2-(methansulfonyl)ethyl]amino}methyl)-2-furyl]4-chinazolinamin-Ditosylat-Monohydrat erst in einer Beladungsdosis über 1 bis 3 Tage, gefolgt von der Verabreichung der Erhaltungsdosis der Verbindung, verabreicht wird, oder die Verbindung *N*-{(1*S*)-2-Amino-1-[(3-fluorphenyl)methyl]ethyl}-5-chlor-4-(4-chlor-1-methyl-1*H*-pyrazol-5-yl)-2-thiophencarboxamid-Hydrochlorid zuerst in einer Beladungsdosis über 1 bis 3 Tage, gefolgt von der Verabreichung der Erhaltungsdosis der Verbindung, verabreicht wird.

**14.** Verwendung nach Anspruch 5, oder Kombination oder Kombinationskit zur Verwendung nach einem der Ansprüche 4 und 6 bis 13, wobei der Krebs entweder Ras/Raf-, PIK3CA/PTEN-, AKT-, EGFR- oder ErbB-2-Wildtyp oder -Mutante ist oder Amplifikation der PIK3CA-, AKT-, EGFR- oder ErbB-2-Gene aufweist oder Überexpression des EGFR- oder ErbB2-Proteins aufweist.

**15.** Verwendung nach Anspruch 5, oder Kombination oder Kombinationskit zur Verwendung nach einem der Ansprüche 4 und 6 bis 13, wobei der Krebs aus Eierstockkrebs, Brustkrebs, Pankreaskrebs oder Prostatakrebs ausgewählt ist.

**Revendications**

**1.** Combinaison comprenant :

(i) un composé de structure (I) :

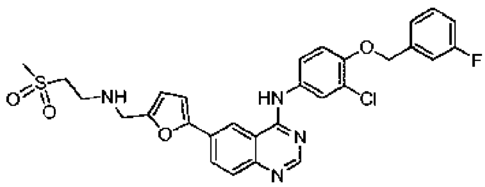

**(I);**

ou un hydrate et/ou sel pharmaceutiquement acceptable de celui-ci ; et
(ii) un composé de structure (II) :

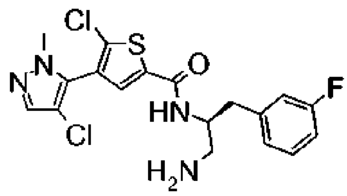

(II)

ou un sel pharmaceutiquement acceptable de celui-ci.

2. Combinaison selon la revendication 1 dans laquelle le composé de structure (I) est sous la forme d'un sel de ditosylate monohydraté et le composé de structure (II) est sous la forme d'un sel de chlorhydrate.

3. Trousse de combinaison comprenant une combinaison selon la revendication 1 ou la revendication 2 conjointement avec un véhicule ou des véhicules pharmaceutiquement acceptables.

4. Combinaison selon la revendication 1 ou la revendication 2 ou trousse de combinaison selon la revendication 3 dans laquelle la quantité du composé de structure (I) est une quantité choisie parmi 750 mg à 1 250 mg, et la quantité du composé de structure (II) est une quantité choisie parmi 50 mg à 300 mg.

5. Utilisation d'une combinaison selon la revendication 1 ou la revendication 2 ou d'une trousse de combinaison selon la revendication 3 ou d'une combinaison ou trousse de combinaison selon la revendication 4 dans la fabrication d'un médicament ou de médicaments pour le traitement du cancer.

6. Combinaison ou trousse de combinaison selon l'une quelconque des revendications 1 à 4 pour utilisation dans le traitement du cancer.

7. Combinaison ou trousse de combinaison pour utilisation dans le traitement du cancer, comprenant une quantité thérapeutiquement efficace d'une combinaison de N-{3-chloro-4-[(3-fluorobenzyl)oxy]phényl}-6-[5-({[2-(méthanesulfonyl)éthyl]amino}méthyl)-2-furyl]-4-quinazolinamine, ou un hydrate et/ou sel pharmaceutiquement acceptable de celle-ci, et de *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide, ou un sel pharmaceutiquement acceptable de celui-ci,
la combinaison étant administrée dans une période spécifiée, et
la combinaison étant administrée pendant une certaine durée.

8. Combinaison ou trousse de combinaison pour utilisation selon la revendication 7 dans laquelle la quantité de ditosylate de N-{3-chloro-4-[(3-fluorobenzyl)oxy]phényl}-6-[5-({[2-(méthanesulfonyl)éthyl]amino}méthyl)-2-furyl]-4-quinazolinamine monohydraté est choisie parmi environ 750 mg à environ 1 250 mg, et cette quantité est administrée une fois par jour dans un ou plusieurs comprimés, et la quantité de chlorhydrate de *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide est choisie parmi environ 50 mg à environ 300 mg, et cette quantité est administrée une fois par jour.

9. Combinaison ou trousse de combinaison pour utilisation selon la revendication 8, le ditosylate de N-{3-chloro-4-[(3-fluorobenzyl)oxy]phényl}-6-[5-({[2-(méthanesulfonyl)éthyl]amino}méthyl)-2-furyl]-4-quinazolinamine monohydraté et le chlorhydrate de *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide étant administrés à au plus 12 heures d'écart l'un de l'autre pendant 1 à 3 jours consécutifs suivis par l'administration de ditosylate de N-{3-chloro-4-[(3-fluorobenzyl)oxy]phényl}-6-[5-({[2-(méthanesulfonyl)éthyl]amino}méthyl)-2-furyl]-4-quinazolinamine monohydraté pendant 3 à 7 jours consécutifs, facultativement suivie par un ou plusieurs cycles d'administration répétée.

10. Combinaison ou trousse de combinaison pour utilisation selon la revendication 8, le ditosylate de N-{3-chloro-4-[(3-fluorobenzyl)oxy]phényl}-6-[5-({[2-(méthanesulfonyl)éthyl]amino}méthyl)-2-furyl]-4-quinazolinamine monohydraté et le chlorhydrate de *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide, étant administrés pendant au moins 7 jours consécutifs.

11. Combinaison ou trousse de combinaison pour utilisation selon la revendication 7, la N-{3-chloro-4-[(3-fluorobenzyl)oxy]phényl}-6-[5-({[2-(méthanesulfonyl)éthyl]amino}méthyl)-2-furyl]-4-quinazolinamine ou un hydrate et/ou sel pharmaceutiquement acceptable de celle-ci, et le *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide ou un sel pharmaceutiquement acceptable de celui-ci étant administrés à au plus 12 heures d'écart l'un de l'autre pendant au moins 5 jours consécutifs.

**12.** Combinaison ou trousse de combinaison pour utilisation selon la revendication 10, le ditosylate de N-{3-chloro-4-[(3-fluorobenzyl)oxy]phényl}-6-[5-({[2-(méthanesulfonyl)éthyl]amino}méthyl)-2-furyl]-4-quinazolinamine monohydraté et le chlorhydrate de *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide étant administrés pendant au moins 14 jours consécutifs.

**13.** Combinaison ou trousse de combinaison pour utilisation selon la revendication 7 ou la revendication 8, le composé ditosylate de N-{3-chloro-4-[(3-fluorobenzyl)oxy]phényl}-6-[5-({[2-(méthanesulfonyl)éthyl]amino}méthyl)-2-furyl]-4-quinazolinamine monohydraté étant administré en premier à une dose de charge pendant 1 à 3 jours suivi par l'administration d'une dose de maintenance du composé, ou le composé chlorhydrate de *N*-{(1*S*)-2-amino-1-[(3-fluorophényl)méthyl]éthyl}-5-chloro-4-(4-chloro-1-méthyl-1*H*-pyrazol-5-yl)-2-thiophènecarboxamide étant administré en premier à une dose de charge pendant 1 à 3 jours suivie par l'administration d'une dose de maintenance du composé.

**14.** Utilisation selon la revendication 5, ou combinaison ou trousse de combinaison pour utilisation selon l'une quelconque des revendications 4 et 6 à 13, le cancer étant de type sauvage ou mutant pour Ras/Raf, PIK3CA/PTEN, AKT, EGFR ou ErbB-2 ou présente une amplification des gènes PIK3CA, AKT, EGFR ou ErbB-2 ou présente une surexpression de protéine EGFR ou ErbB2.

**15.** Utilisation selon la revendication 5, ou combinaison ou trousse de combinaison pour utilisation selon l'une quelconque des revendications 4 et 6 à 13, le cancer étant choisi parmi un cancer de l'ovaire, du sein, du pancréas et de la prostate.

# Figure 1.

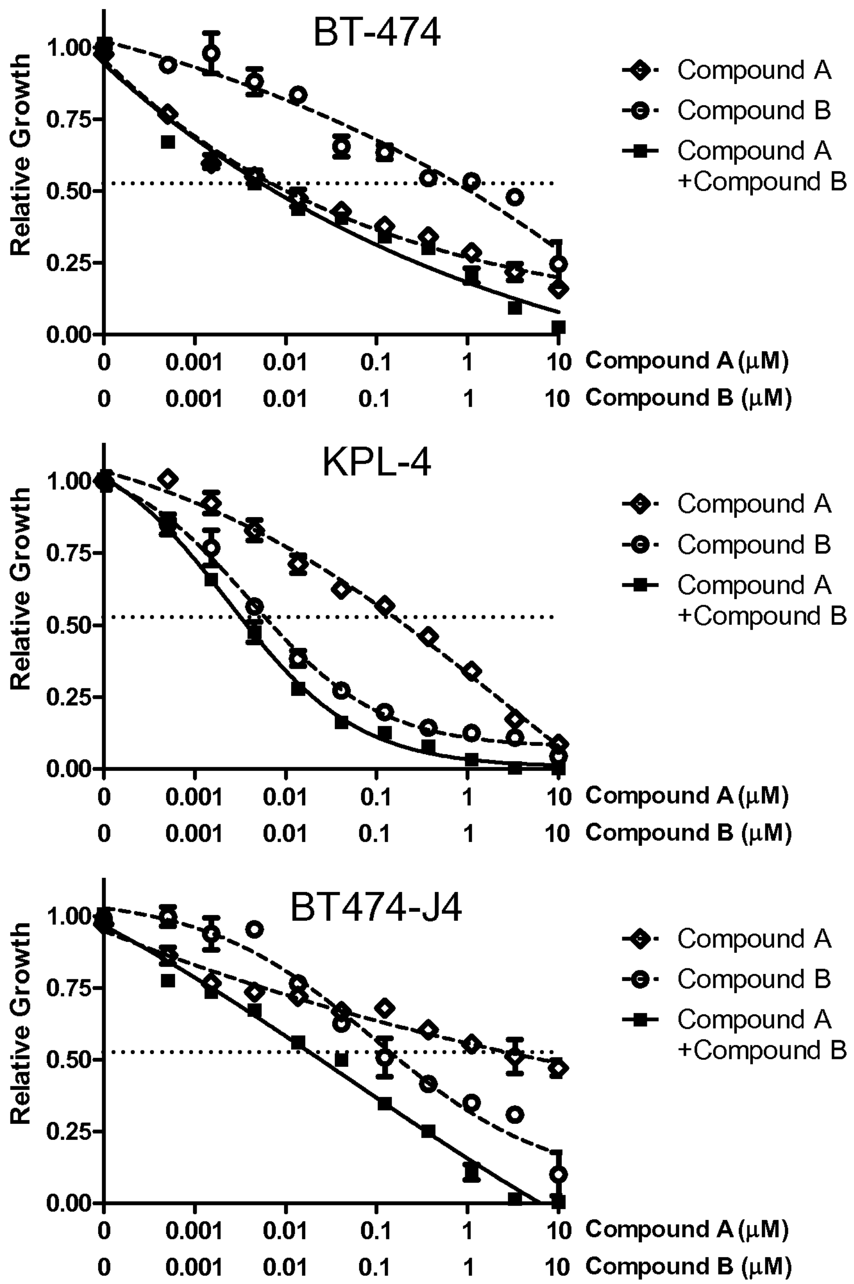
BT-474
Relative Growth
1.00
0.75
0.50
0.25
0.00
Compound A
Compound B
Compound A
+Compound B
0
0.001
0.01
0.1
1
10
Compound A (μM)
0
0.001
0.01
0.1
1
10
Compound B (μM)
KPL-4
Relative Growth
1.00
0.75
0.50
0.25
0.00
Compound A
Compound B
Compound A
+Compound B
0
0.001
0.01
0.1
1
10
Compound A (μM)
0
0.001
0.01
0.1
1
10
Compound B (μM)
BT474-J4
Relative Growth
1.00
0.75
0.50
0.25
0.00
Compound A
Compound B
Compound A
+Compound B
0
0.001
0.01
0.1
1
10
Compound A (μM)
0
0.001
0.01
0.1
1
10
Compound B (μM)

# Figure 2.

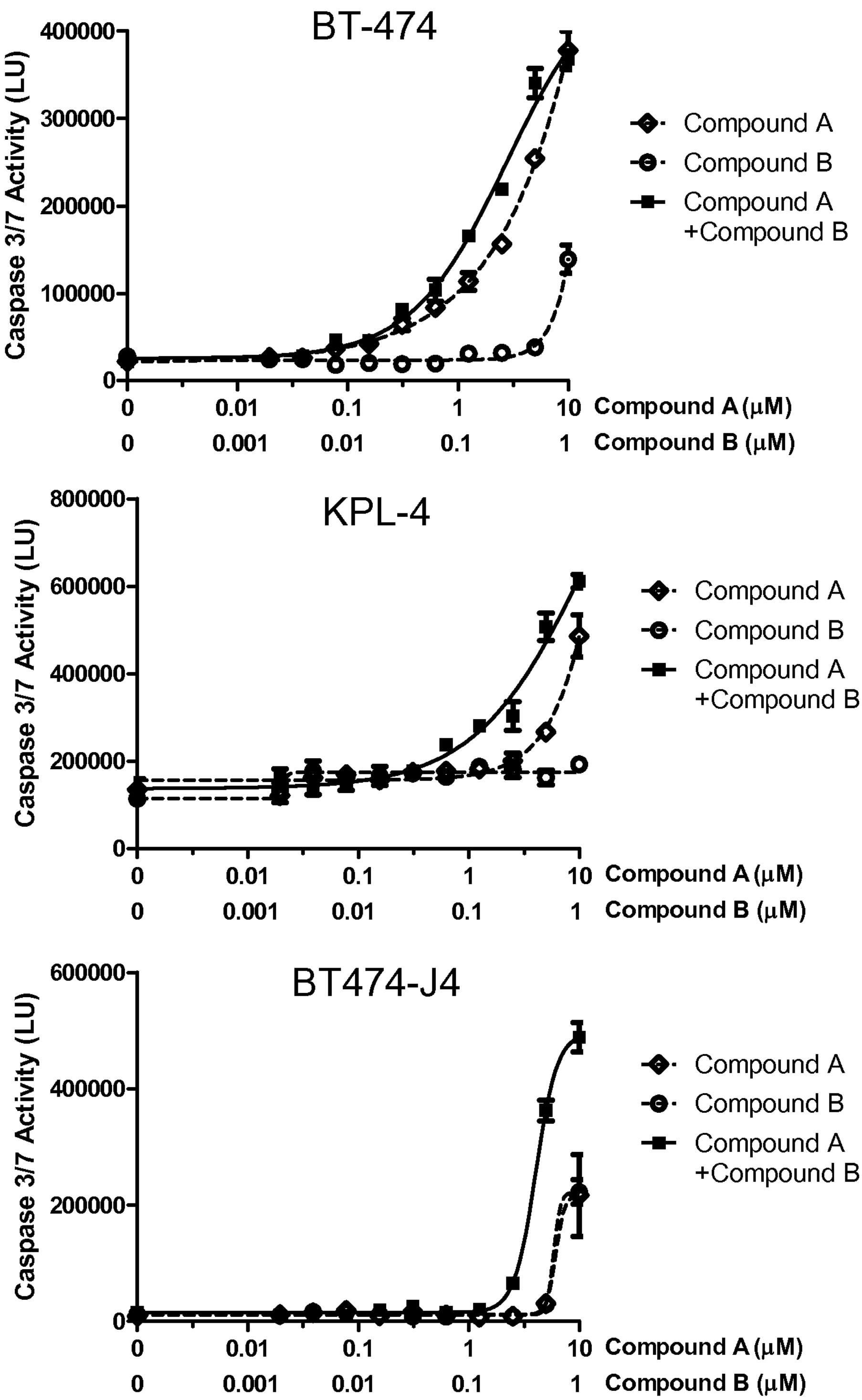
BT-474
Caspase 3/7 Activity (LU)
400000
300000
200000
100000
0
0
0.01
0.1
1
10
Compound A (μM)
0
0.001
0.01
0.1
1
Compound B (μM)
Compound A
Compound B
Compound A +Compound B
KPL-4
Caspase 3/7 Activity (LU)
800000
600000
400000
200000
0
0
0.01
0.1
1
10
Compound A (μM)
0
0.001
0.01
0.1
1
Compound B (μM)
Compound A
Compound B
Compound A +Compound B
BT474-J4
Caspase 3/7 Activity (LU)
600000
400000
200000
0
0
0.01
0.1
1
10
Compound A (μM)
0
0.001
0.01
0.1
1
Compound B (μM)
Compound A
Compound B
Compound A +Compound B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5773476 A
- WO 9935146 A
- WO 2008063853 A
- WO 2011060380 A
- US 2007015775 A
- US 2010041726 A
- EP 9900048 W
- US 0120706 W
- WO 0202552 A
- US 2006014447 W
- WO 06113649 A
- US 2008053269 W
- WO 2008098104 A
- US 2010022323 W
- WO 2010088331 A
- US 7576209 B


### Non-patent literature cited in the description


- **CREWS ; ERIKSON.** *Cell,* 1993, vol. 74, 215-17
- **A.F. WILKS.** *Progress in Growth Factor Research,* 1990, vol. 2, 97-111
- **S.A. COURTNEIDGE.** *Dev.,* 1993, vol. I, 57-64
- **J.A. COOPER.** *Semin. Cell Biol.,* 1994, vol. 5 (6), 377-387
- **R.F. PAULSON.** *Semin. Immunol.,* 1995, vol. 7 (4), 267-277
- **A.C. CHAN.** *Curr. Opin. Immunol.,* 1996, vol. 8 (3), 394-401
- **B.TANNER.** *J Clin Oncol.,* 2006, vol. 24 (26), 4317-23
- **M. HAYASHI.** *Clin. Cancer Res.,* 2008, vol. 14 (23), 7843-9
- **H. KAYA.** *Eur J Gynaecol Oncol.,* 2008, vol. 29 (4), 350-6
- **M.C. HUNG et al.** *Seminars in Oncology,* August 1999, vol. 26 (4), 51-59
- **ULLRICH et al.** *Cell,* 20 April 1990, vol. 61, 203-212
- **MODJTAHEDI et al.** *Int'l. J. of Oncology,* 1998, vol. 13, 335-342
- **J.R. WOODBURN.** *Pharmacol. Ther.,* 1999, vol. 82 (2-3), 241-250
- **ADAMS et al.** *Science,* 1998, vol. 281, 1322-1326
- **THORNBERRY et al.** *Science,* 1998, vol. 281, 1312-1316
- **KULIK et al.** *Mol.Cell.Biol.,* 1997, vol. 17, 1595-1606
- **FRANKE et al.** *Cell,* 1997, vol. 88, 435-437
- **KAUFFMANN-ZEH et al.** *Nature,* 1997, vol. 385, 544-548
- **HEMMINGS.** *Science,* 1997, vol. 275, 628-630
- **DUDEK et al.** *Science,* 1997, vol. 275, 661-665
- **FRANKE et al.** *Cell,* 1995, vol. 81, 727-736
- **HEMMINGS.** *Science,* 1997, vol. 277, 534
- **DOWNWARD.** *Curr. Opin. Cell Biol.,* 1998, vol. 10, 262-267
- **ALESSI et al.** *EMBO J.,* 1996, vol. 15, 6541-6551
- **J. Q. CHEUNG et al.** *Proc. Natl. Acad. Sci. U.S.A.,* 1992, vol. 89, 9267-9271
- **J. Q. CHEUNG et al.** *Proc. Natl. Acad. Sci. U.S.A.,* 1996, vol. 93, 3636-3641
- **NAKATANI et al.** *J. Biol.Chem.,* 1999, vol. 274, 21528-21532
- **BELLACOSA et al.** *Int. J. Cancer,* 1995, vol. 64, 280-285
- **SUN et al.** *Am. J. Pathol.,* 2001, vol. 159, 431-7
- **LI et al.** *Science,* 1997, vol. 275, 1943-1947
- **STAMBOLIC et al.** *Cell,* 1998, vol. 95, 29-39
- **SUN et al.** *Proc. Nati. Acad. Sci. U.S.A.,* 1999, vol. 96, 6199-6204
- **LIAW et al.** *Nature Genetics,* 1997, vol. 16, 64-67
- **GULDBERG et al.** *Cancer Research,* 1997, vol. 57, 3660-3663
- **RISINGER et al.** *Cancer Research,* 1997, vol. 57, 4736-4738
- **KORKOLA et al.** *Mol. Cancer Ther.,* 2009, vol. 8, A179
- **HIRAI et al.** *Mol. Cancer Ther.,* June 2010, vol. 9, 1956-1967
- **CHOU TC ; TALALAY P.** Quantitative analysis of dose-effect relationships: the combined effects of multiple drugs or enzyme inhibitors. *Adv Enzyme Regul,* 1984, vol. 22, 27-55

- **LIU L ; SHI H ; LIU Y ; ANDERSON A ; PETERSON J ; GREGER J ; MARTIN AM ; GILMER TM.** Synergistic Effects of Foretinib with HER-Targeted Agents in MET and HER1- or HER2-Coactivated Tumor Cells. *Mol Cancer Ther.,* 2011, vol. 10, 518-30
- **TANNER et al.** *Mol Cancer Ther,* 2004, vol. 3, 1585-92